(19)

**Europäisches Patentamt**
**European Patent Office**
**Office européen des brevets**

(11) **EP 4 124 661 A1**

(12) **EUROPEAN PATENT APPLICATION**

(43) Date of publication:
**01.02.2023 Bulletin 2023/05**

(21) Application number: **21187665.1**

(22) Date of filing: **26.07.2021**

(51) International Patent Classification (IPC):
***C12Q 1/6886*** (2018.01)

(52) Cooperative Patent Classification (CPC):
**C12Q 1/6886**; C12Q 2600/106; C12Q 2600/158

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Koninklijke Philips N.V.**
**5656 AG Eindhoven (NL)**
• **The University Court of the University of Glasgow**
**Glasgow G12 8QQ (GB)**

(72) Inventors:
• **HOFFMANN, Ralf Dieter**
**5656 AE Eindhoven (NL)**
• **BAILLIE, George**
**5656 AE Eindhoven (NL)**

(74) Representative: **Philips Intellectual Property & Standards**
**High Tech Campus 52**
**5656 AG Eindhoven (NL)**

Remarks:
The complete document including Reference Table(s) and the Sequence Listing(s) can be downloaded from the EPO website

(54) **PERSONALIZATION IN PROSTATE CANCER BY USE OF THE PROSTATE CANCER PDE4D7 KNOCK-DOWN SCORE**

(57) The invention relates to a method of predicting a response of a prostate cancer subject to radiotherapy, comprising determining or receiving the result of a determination of a gene expression profile for each of three or more PDE4D7 knockdown differentially expressed genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, said gene expression profiles being determined in a biological sample obtained from the subject, and determining the prediction of the radiotherapy response based on the gene expression profiles for the three or more PDE4D7 knockdown genes.

Fig. 29

EP 4 124 661 A1

**Description**

FIELD OF THE INVENTION

The invention relates to a method of predicting a response of a prostate cancer subject to radiotherapy, and to a computer program product for predicting a response of a prostate cancer subject to radiotherapy. Moreover, the invention relates to a diagnostic kit, to a use of the kit, to a use of the kit in a method of predicting a response of a prostate cancer subject to radiotherapy, to a use of a gene expression profile for each of one or more PDE4D7 knockdown responsive genes in a method of predicting a response of a prostate cancer subject to radiotherapy, and to a corresponding computer program product.

BACKGROUND OF THE INVENTION

Cancer is a class of diseases in which a group of cells displays uncontrolled growth, invasion and sometimes metastasis. These three malignant properties of cancers differentiate them from benign tumours, which are self-limited and do not invade or metastasize. Prostate Cancer (PCa) is the second most commonly-occurring non-skin malignancy in men, with an estimated 1.3 million new cases diagnosed and 360,000 deaths world-wide in 2018 (see Bray F. et al., "Global cancer statistics 2018: GLOBOCAN estimates of incidence and mortality worldwide for 36 cancers in 185 countries", CA Cancer J Clin, Vol. 68, No. 6, pages 394-424, 2018). In the US, about 90% of the new cases concern localized cancer, meaning that metastases have not yet been formed (see ACS (American Cancer Society), "Cancer Facts & Figures 2010", 2010).
For the treatment of primary localized prostate cancer, several radical therapies are available, of which surgery (radical prostatectomy, RP) and radiation therapy (RT) are most commonly used. RT is administered via an external beam or via the implantation of radioactive seeds into the prostate (brachytherapy) or a combination of both. It is especially preferable for patients who are not eligible for surgery or have been diagnosed with a tumour in an advanced localized or regional stage. Radical RT is provided to up to 50% of patients diagnosed with localized prostate cancer in the US (see ACS, 2010, ibid).
After treatment, prostate cancer antigen (PSA) levels in the blood are measured for disease monitoring. An increase of the blood PSA level provides a biochemical surrogate measure for cancer recurrence or progression. However, the variation in reported biochemical progression-free survival (bPFS) is large (see Grimm P. et al., "Comparative analysis of prostate-specific antigen free survival outcomes for patients with low, intermediate and high risk prostate cancer treatment by radical therapy. Results from the Prostate Cancer Results Study Group", BJU Int, Suppl. 1, pages 22-29, 2012). For many patients, the bPFS at 5 or even 10 years after radical RT may lie above 90%. Unfortunately, for the group of patients at medium and especially higher risk of recurrence, the bPFS can drop to around 40% at 5 years, depending on the type of RT used (see Grimm P. et al., 2012, ibid).
A large number of the patients with primary localized prostate cancer that are not treated with RT will undergo RP (see ACS, 2010, ibid). After RP, an average of 60% of patients in the highest risk group experience biochemical recurrence after 5 and 10 years (see Grimm P. et al., 2012, ibid). In case of biochemical progression after RP, one of the main challenges is the uncertainty whether this is due to recurring localized disease, one or more metastases or even an indolent disease that will not lead to clinical disease progression (see Dal Pra A. et al., "Contemporary role of postoperative radiotherapy for prostate cancer", Transl Androl Urol, Vo. 7, No. 3, pages 399-413, 2018, and Herrera F.G. and Berthold D.R., "Radiation therapy after radical prostatectomy: Implications for clinicians", Front Oncol, Vol. 6, No. 117, 2016). RT to eradicate remaining cancer cells in the prostate bed is one of the main treatment options to salvage survival after a PSA increase following RP. The effectiveness of salvage radiotherapy (SRT) results in 5-year bPFS for 18% to 90% of patients, depending on multiple factors (see Herrera F.G. and Berthold D.R., 2016, ibid, and Pisansky T.M. et al., "Salvage radiation therapy dose response for biochemical failure of prostate cancer after prostatectomy - A multi-institutional observational study", Int J Radiat Oncol Biol Phys, Vol. 96, No. 5, pages 1046-1053, 2016).
It is clear that for certain patient groups, radical or salvage RT is not effective. Their situation is even worsened by the serious side effects that RT can cause, such as bowel inflammation and dysfunction, urinary incontinence and erectile dysfunction (see Resnick M.J. et al., "Long-term functional outcomes after treatment for localized prostate cancer", N Engl J Med, Vol. 368, No. 5, pages 436-445, 2013, and Hegarty S.E. et al., "Radiation therapy after radical prostatectomy for prostate cancer: Evaluation of complications and influence of radiation timing on outcomes in a large, population-based cohort", PLoS One, Vol. 10, No. 2, 2015). In addition, the median cost of one course of RT based on Medicare reimbursement is $ 18,000, with a wide variation up to about $ 40,000 (see Paravati A.J. et al., "Variation in the cost of radiation therapy among medicare patients with cancer", J Oncol Pract, Vol. 11, No. 5, pages 403-409, 2015). These figures do not include the considerable longitudinal costs of follow-up care after radical and salvage RT.
An improved prediction of effectiveness of RT for each patient, be it in the radical or the salvage setting, would improve therapy selection and potentially survival. This can be achieved by 1) optimizing RT for those patients where RT is predicted to be effective (e.g., by dose escalation or a different starting time) and 2) guiding patients where RT is predicted not to be effective to an alternative, potentially more effective form of treatment. Further, this would reduce suffering for those patients who would be spared ineffective therapy and would reduce costs spent on ineffective therapies.

Numerous investigations have been conducted into measures for response prediction of radical RT (see Hall W.A. et al., "Biomarkers of outcome in patients with localized prostate cancer treated with radiotherapy", Semin Radiat Oncol, Vol. 27, pages 11-20, 2016, and Raymond E. et al., "An appraisal of analytical tools used in predicting clinical outcomes following radiation therapy treatment of men with prostate cancer: A systematic review", Radiat Oncol, Vol. 12, No. 1, page 56, 2017) and SRT (see Herrera F.G. and Berthold D.R., 2016, ibid). Many of these measures depend on the concentration of the blood-based biomarker PSA. Metrics investigated for prediction of response before start of RT (radical as well as salvage) include the absolute value of the PSA concentration, its absolute value relative to the prostate volume, the absolute increase over a certain time and the doubling time. Other frequently considered factors are the Gleason score and the clinical tumour stage. For the SRT setting, additional factors are relevant, e.g., surgical margin status, time to recurrence after RP, pre-/peri-surgical PSA values and clinico-pathological parameters.

Although these clinical variables provide limited improvements in patient stratification in various risk groups, there is a need for better predictive tools.

A wide range of biomarker candidates in tissue and bodily fluids has been investigated, but validation is often limited and generally demonstrates prognostic information and not a predictive (therapy-specific) value (see Hall W.A. et al., 2016, ibid). A small number of gene expression panels is currently being validated by commercial organizations. One or a few of these may show predictive value for RT in future (see Dal Pra A. et al., 2018, ibid).

In conclusion, a strong need for better prediction of response to RT remains, for primary prostate cancer as well as for the post-surgery setting.

SUMMARY OF THE INVENTION

In a first aspect, the invention relates to a method of predicting a response of a prostate cancer subject to radiotherapy, comprising: determining or receiving the result of a determination of the gene expression levels for each of three or more genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, said gene expression levels being determined in a biological sample obtained from the subject, determining the prediction of the radiotherapy response based on the gene expression levels for the three or more genes, wherein said prediction is a favorable repsonse or a non-favorable response to radiotherapy.

In a second aspect, the invention relates to a computer program product comprising instructions which, when the program is executed by a computer, cause the computer to carry out a method comprising: receiving data indicative of a gene expression profile for each of three or more, for example, 3, 4, 5, 6, 7, 8, 9 or all, genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, said gene expression levels being determined in a biological sample obtained from a prostate cancer subject, determining a prediction of a response of a prostate cancer subject to therapy based on the gene expression profile(s) for the three or more genes, wherein said prediction is a favorable repsonse or a non-favorable response to radiotherapy.

In a third aspect, the invention relates to a diagnostic kit, comprising: at least three sets of polymerase chain reaction primer pair, and optionally at least three probes, for determining the expression level for each of three or more, for example, 3, 4, 5, 6, 7, 8, 9 or all, genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, in a sample.

In a fourth aspect, the invention relates to use of the kit as defined in third aspect of the invention in a method of predicting a response of a prostate cancer subject to radiotherapy, preferably for use in the method as defined in the first aspect of the invention.

In a fifth aspect, the invention relates to a method, comprising: receiving a biological sample obtained from a prostate cancer subject, using the kit as defined in claim 12 to determine a gene expression profile for each of three or more, for example, 3, 4, 5, 6, 7, 8, 9 or all, genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, in the biological sample obtained from the subject.

BRIEF DESCRIPTION OF THE DRAWINGS

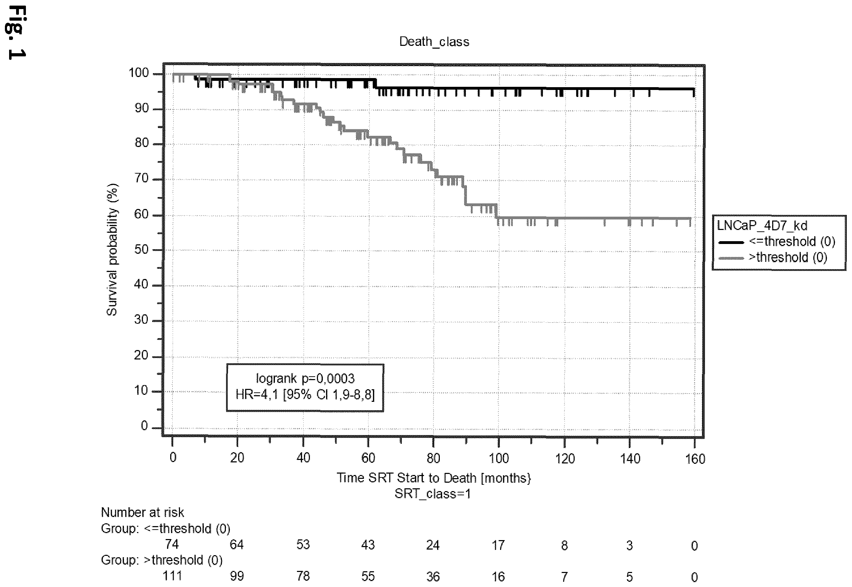

Fig. 1 shows a Kaplan-Meier curve of the PDE4D7_KD_model in a 185 patient cohort (training set used to develop the PDE4D7_KD_model) with all patients undergoing SRT (salvage radiation treatment) after post-surgical BCR (biochemical recurrence). The clinical endpoint that was tested was overall survival (Death) after the start of salvage radiation therapy (SRT) due to post-surgical disease recurrence. Logrank, HR and confidence interval are indicated in the figure. The included supplementary lists indicate the number of patients at risk for the PDE4D7_KD_model classes analyzed, i.e., the patients at risk at any time interval +20 months after surgery are shown.

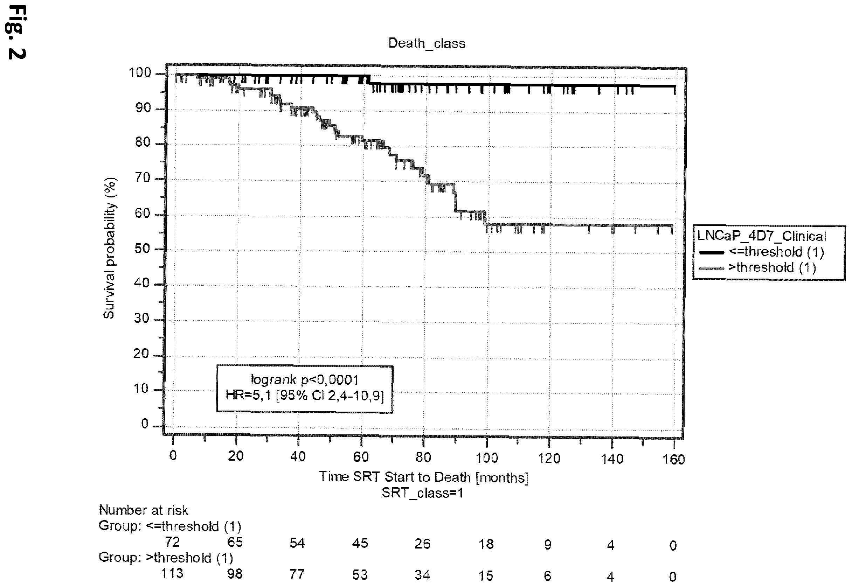

Fig. 2 shows a Kaplan-Meier curve of the PDE4D7_clinical_model in a 185 patient cohort (training set used to develop the PDE4D7_clinical_model) with all patients undergoing SRT (salvage radiation treatment) after post-surgical BCR (biochemical recurrence). The clinical endpoint that was tested was overall survival (Death) after the start of salvage radiation therapy (SRT) due to post-surgical disease recurrence. Logrank, HR and confidence interval are indicated in the figure. The included supplementary lists indicate the number of patients at risk for the PDE4D7_clinical_model classes analyzed, i.e., the patients at risk at any time interval +20 months after surgery are shown.

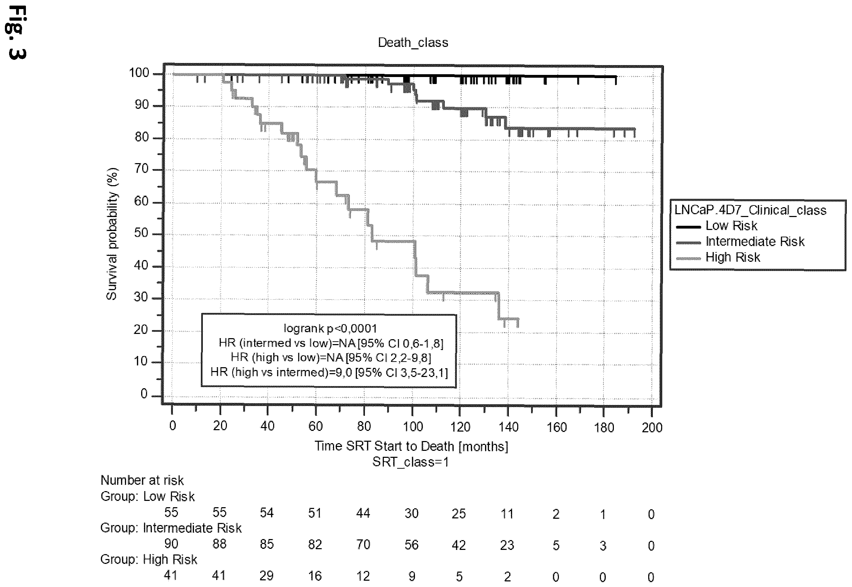

Fig. 3 shows a Kaplan-Meier curve of the PDE4D7_clinical class_model in a 185 patient cohort (training set used to develop the PDE4D7_clinical class model) with all patients undergoing SRT (salvage radiation treatment) after post-surgical BCR (biochemical recurrence). The clinical endpoint that was tested was overall survival (Death) after the start of salvage radiation therapy (SRT) due to post-surgical disease recurrence. Logrank, HR and confidence interval are indicated in the figure. The included supplementary lists indicate the number of patients at risk for the PDE4D7_clinical class_model classes analyzed, i.e., the patients at risk at any time interval +20 months after surgery are shown.

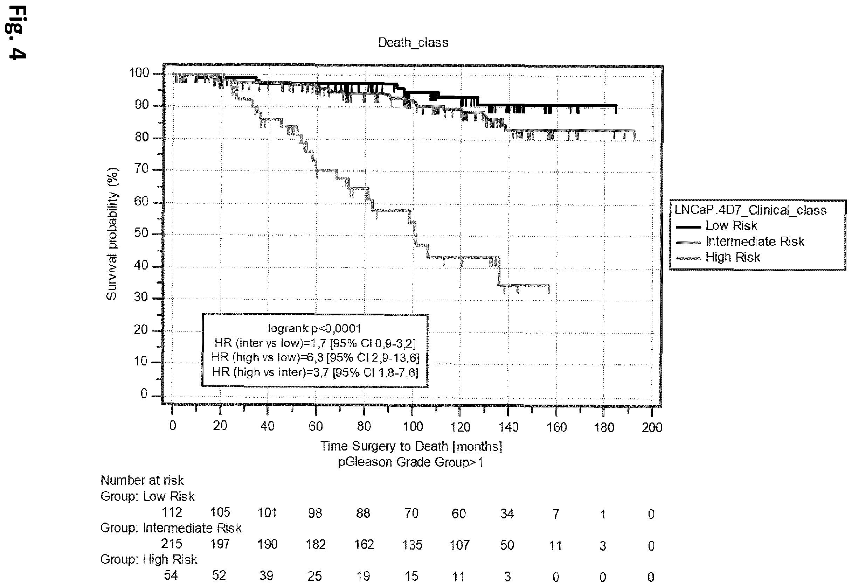

Fig. 4 shows a Kaplan-Meier curve of the PDE4D7_clinical class_model in a 381 patient cohort (training set used to develop the PDE4D7_clinical class model) with all patients undergoing SRT (salvage radiation treatment) after post-surgical BCR (biochemical recurrence). The clinical endpoint that was tested was overall survival (Death) after the start of salvage radiation therapy (SRT) due to post-surgical disease recurrence. Logrank, HR and confidence interval are indicated in the figure. The included supplementary lists indicate the number of patients at risk for the PDE4D7_clinical class_model classes analyzed, i.e., the patients at risk at any time interval +20 months after surgery are shown.

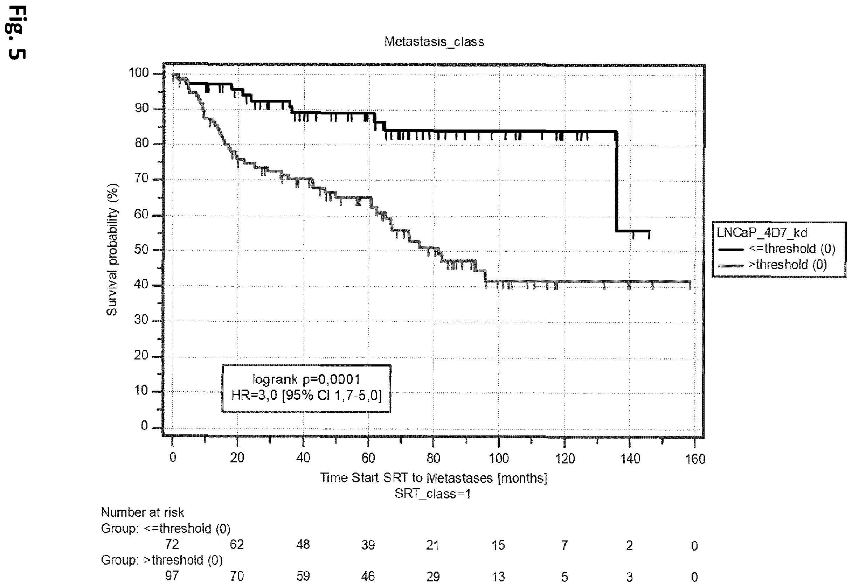

Fig. 5 shows a Kaplan-Meier curve of the PDE4D7_KD_model in a 169 patient cohort (training set used to develop the PDE4D7_KD_model) with all patients undergoing SRT (salvage radiation treatment) after post-surgical BCR (biochemical recurrence). The clinical endpoint that was tested was metastases progression free survival (metastasis) after the start of salvage radiation therapy (SRT) due to post-surgical disease recurrence. Logrank, HR and confidence interval are indicated in the figure. The included supplementary lists indicate the number of patients at risk for the PDE4D7_KD_model classes analyzed, i.e., the patients at risk at any time interval +20 months after surgery are shown.

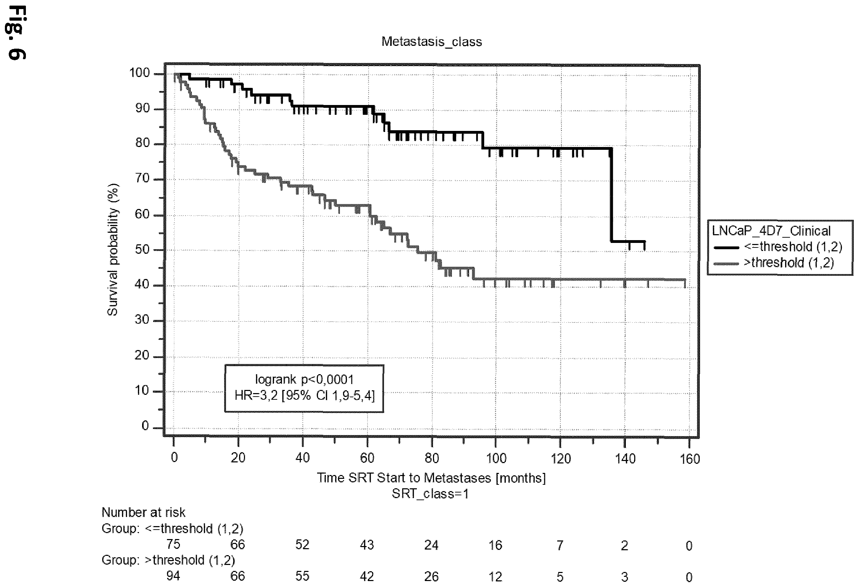

Fig. 6 shows a Kaplan-Meier curve of the PDE4D7_clinical_model in a 169 patient cohort (training set used to develop the PDE4D7_clinical_model) with all patients undergoing SRT (salvage radiation treatment) after post-surgical BCR (biochemical recurrence). The clinical endpoint that was tested was metastases progression free survival (metastasis) after the start of salvage radiation therapy (SRT) due to post-surgical disease recurrence. Logrank, HR and confidence interval are indicated in the figure. The included supplementary lists indicate the number of patients at risk for the PDE4D7_clinical_model classes analyzed, i.e., the patients at risk at any time interval +20 months after surgery are shown.

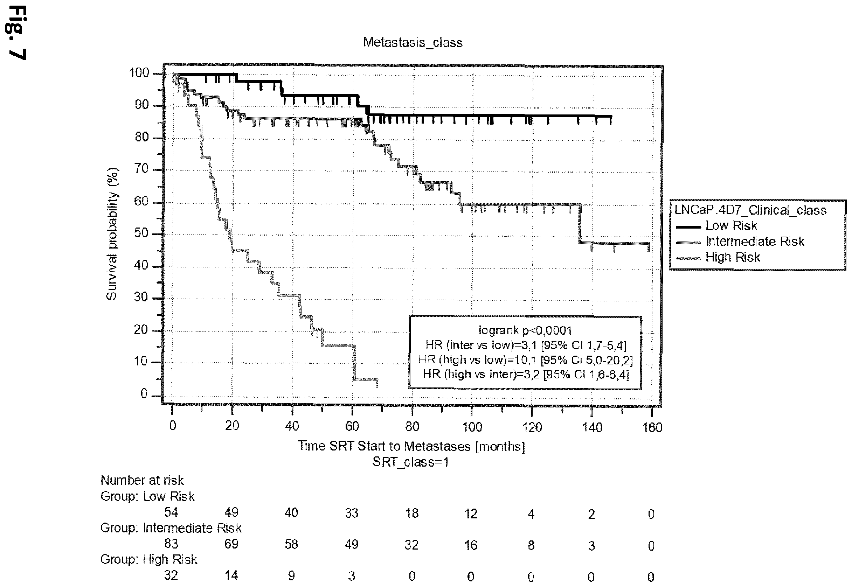

Fig. 7 shows a Kaplan-Meier curve of the PDE4D7_clinical class_model in a 169 patient cohort (training set used to develop the PDE4D7_clinical class model) with all patients undergoing SRT (salvage radiation treatment) after post-surgical BCR (biochemical recurrence). The clinical endpoint that was tested was metastases progression free survival (metastasis) after the start of salvage radiation therapy (SRT) due to post-surgical disease recurrence. Logrank, HR and confidence interval are indicated in the figure. The included supplementary lists indicate the number of patients at risk for the PDE4D7_clinical class_model classes analyzed, i.e., the patients at risk at any time interval +20 months after surgery are shown.

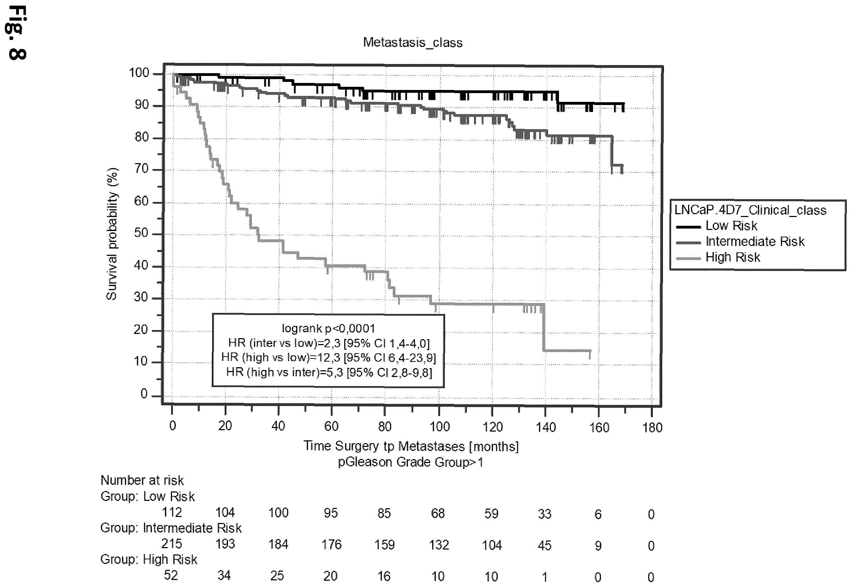

Fig. 8 shows a Kaplan-Meier curve of the PDE4D7_clinical class_model in a 379 patient cohort (training set used to develop the PDE4D7_clinical class model) with all patients undergoing SRT (salvage radiation treatment) after post-surgical BCR (biochemical recurrence). The clinical endpoint that was tested was metastases progression free survival (metastasis) after the start of salvage radiation therapy (SRT) due to post-surgical disease recurrence. Logrank, HR and confidence interval are indicated in the figure. The included supplementary lists indicate the number of patients at risk for the PDE4D7_clinical class_model classes analyzed, i.e., the patients at risk at any time interval +20 months after surgery are shown.

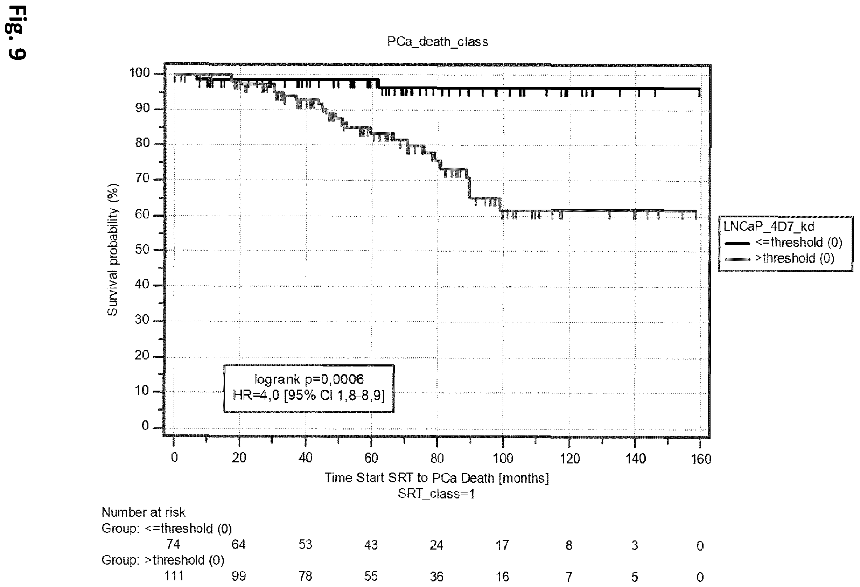

Fig. 9 shows a Kaplan-Meier curve of the PDE4D7_KD_model in a 185 patient cohort (training set used to develop the PDE4D7_KD_model) with all patients undergoing SRT (salvage radiation treatment) after post-surgical BCR (biochemical recurrence). The clinical endpoint that was tested was prostate cancer specific survival (PCa Death) after the start of salvage radiation therapy (SRT) due to post-surgical disease recurrence. Logrank, HR and confidence interval are indicated in the figure. The included supplementary lists indicate the number of patients at risk for the PDE4D7_KD_model classes analyzed, i.e., the patients at risk at any time interval +20 months after surgery are shown.

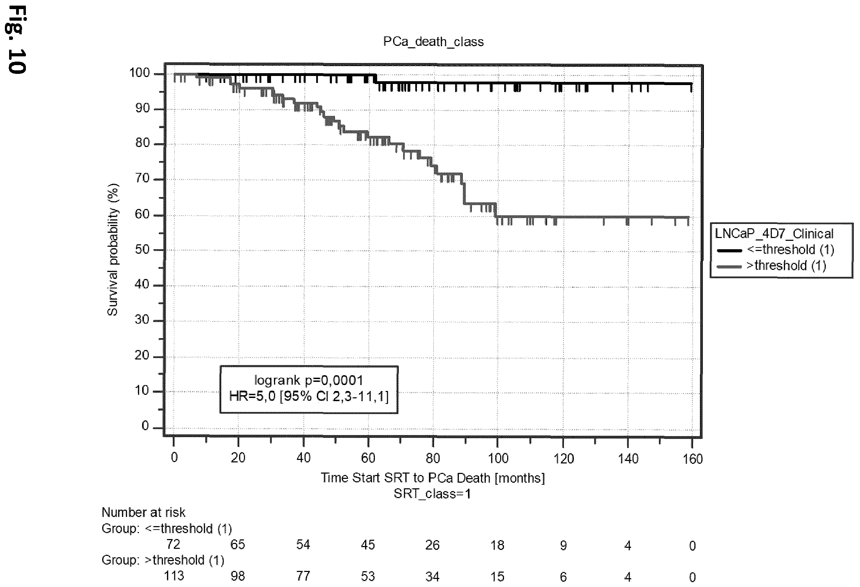

Fig. 10 shows a Kaplan-Meier curve of the PDE4D7_clinical_model in a 185 patient cohort (training set used to develop the PDE4D7_clinical_model) with all patients undergoing SRT (salvage radiation treatment) after post-surgical BCR (biochemical recurrence). The clinical endpoint that was tested was prostate cancer specific survival (PCa Death) after the start of salvage radiation therapy (SRT) due to post-surgical disease recurrence. Logrank, HR and confidence interval are indicated in the figure. The included supplementary lists indicate the number of patients at risk for the PDE4D7_clinical_model classes analyzed, i.e., the patients at risk at any time interval +20 months after surgery are shown.

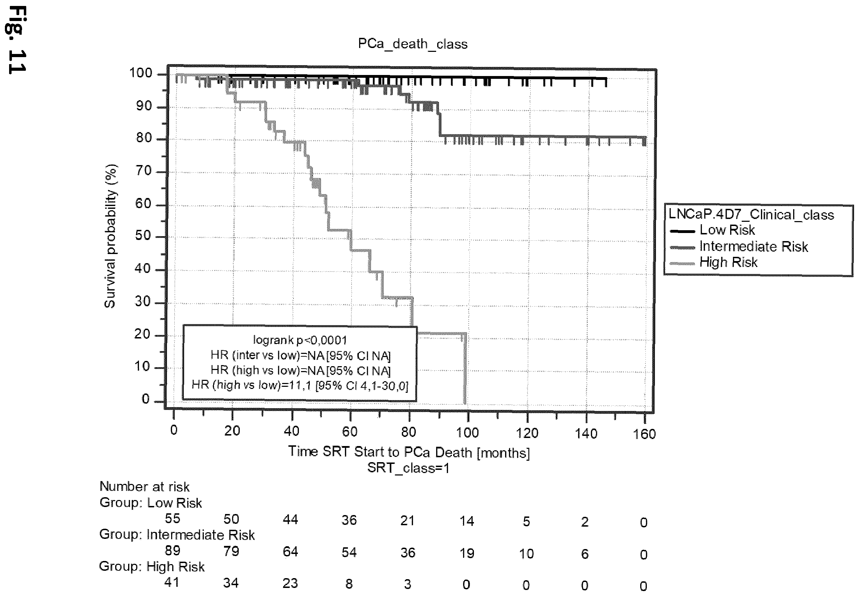

Fig. 11 shows a Kaplan-Meier curve of the PDE4D7_clinical class_model in a 185 patient cohort (training set used to develop the PDE4D7_clinical class model) with all patients undergoing SRT (salvage radiation treatment) after post-surgical BCR (biochemical recurrence). The clinical endpoint that was tested was prostate cancer specific survival (PCa Death) after the start of salvage radiation therapy (SRT) due to post-surgical disease recurrence. Logrank, HR and confidence interval are indicated in the figure. The included supplementary lists indicate the number of patients at risk for the PDE4D7_clinical class_model classes analyzed, i.e., the patients at risk at any time interval +20 months after surgery are shown.

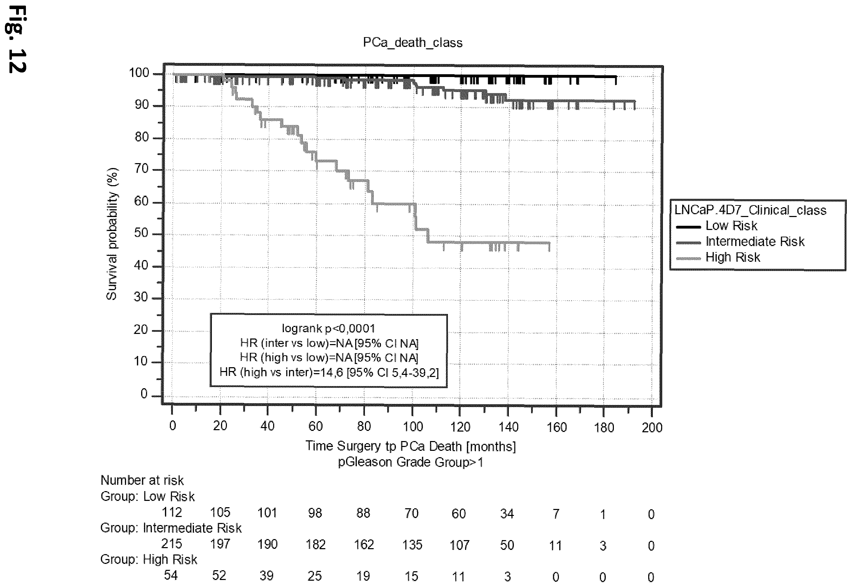

Fig. 12 shows a Kaplan-Meier curve of the PDE4D7_clinical class_model in a 381 patient cohort (training set used to develop the PDE4D7_clinical class model) with all patients undergoing SRT (salvage radiation treatment) after post-surgical BCR (biochemical recurrence). The clinical endpoint that was tested was prostate cancer specific survival (PCa Death) after the start of salvage radiation therapy (SRT) due to post-surgical disease recurrence. Logrank, HR and confidence interval are indicated in the figure. The included supplementary lists indicate the number of patients at risk for the PDE4D7_clinical class_model classes analyzed, i.e., the patients at risk at any time interval +20 months after surgery are shown.

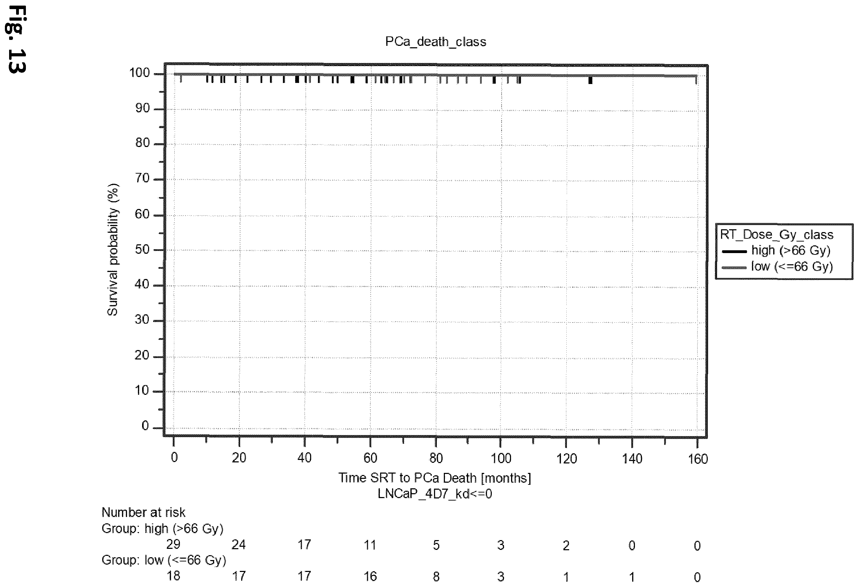

Fig. 13 shows a Kaplan-Meier curve of the PDE4D7_Dose Gy class_model in a 47 patient cohort (training set used to develop the PDE4D7_Dose Gy class model) with all patients undergoing SRT (salvage radiation treatment) after post-surgical BCR (biochemical recurrence). The clinical endpoint that was tested was prostate cancer specific survival (PCa Death) after the start of salvage radiation therapy (SRT) due to post-surgical disease recurrence for subjects receiving a high (> 66 Gy) or low (<= 66 Gy) radiotherapy dosage. Logrank, HR and confidence interval are indicated in the figure. The included supplementary lists indicate the number of patients at risk for the PDE4D7_dose Gy class_model classes analyzed, i.e., the patients at risk at any time interval +20 months after surgery are shown.

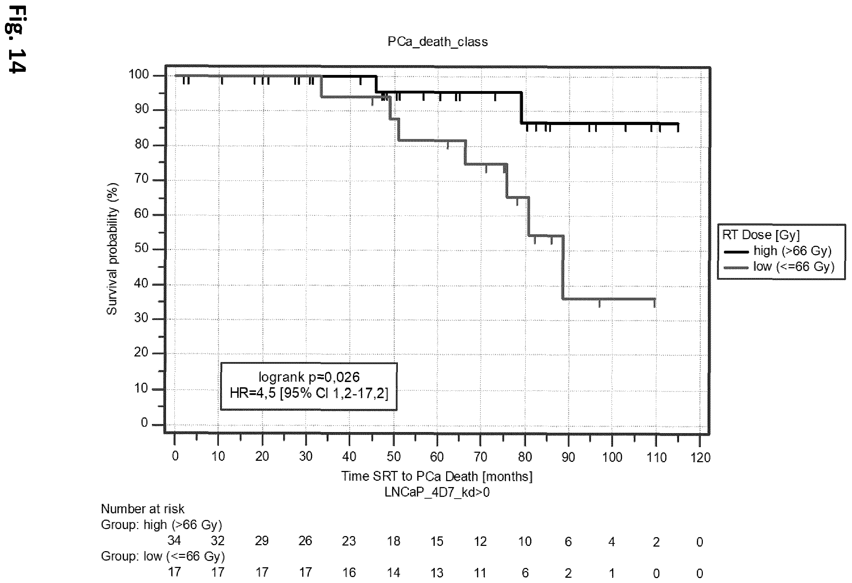

Fig. 14 shows a Kaplan-Meier curve of the PDE4D7_dose Gy class_model in a 51 patient cohort (training set used to develop the PDE4D7_dose Gy class model) with all patients undergoing SRT (salvage radiation treatment) after post-surgical BCR (biochemical recurrence). The clinical endpoint that was tested was prostate cancer specific survival (PCa Death) after the start of salvage radiation therapy (SRT) due to post-surgical disease recurrence for subjects receiving a high (> 66 Gy) or low (<= 66 Gy) radiotherapy dosage. Logrank, HR and confidence interval are indicated in the figure. The included supplementary lists indicate the number of patients at risk for the PDE4D7_dose Gy class_model classes analyzed, i.e., the patients at risk at any time interval +20 months after surgery are shown.

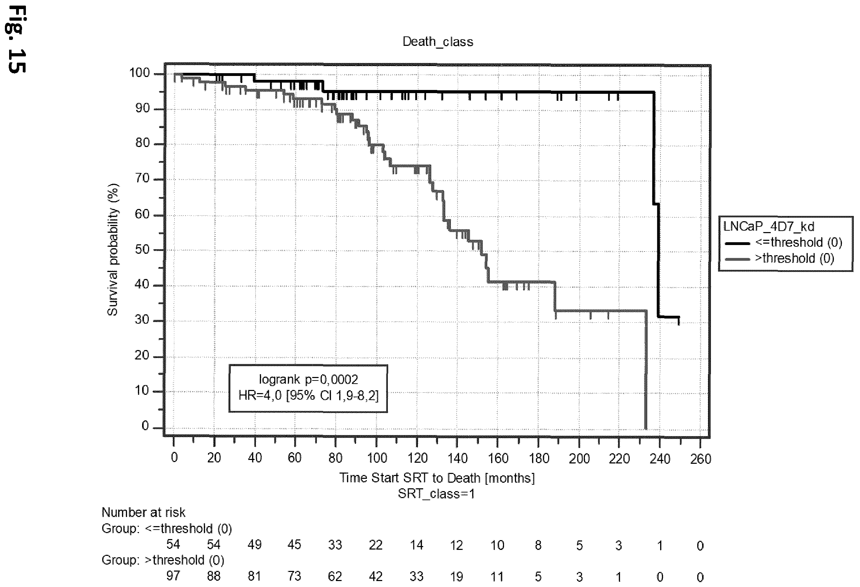

Fig. 15 shows a Kaplan-Meier curve of the PDE4D7_KD_model in a 151 patient cohort (testing set used to validate the PDE4D7_KD_model as developed on the training set) with all patients undergoing SRT (salvage radiation treatment) after post-surgical BCR (biochemical recurrence). The clinical endpoint that was tested was overall survival (Death) after the start of salvage radiation therapy (SRT) due to post-surgical disease recurrence. Logrank, HR and confidence interval are indicated in the figure. The included supplementary lists indicate the number of patients at risk for the PDE4D7_KD_model classes analyzed, i.e., the patients at risk at any time interval +20 months after surgery are shown.

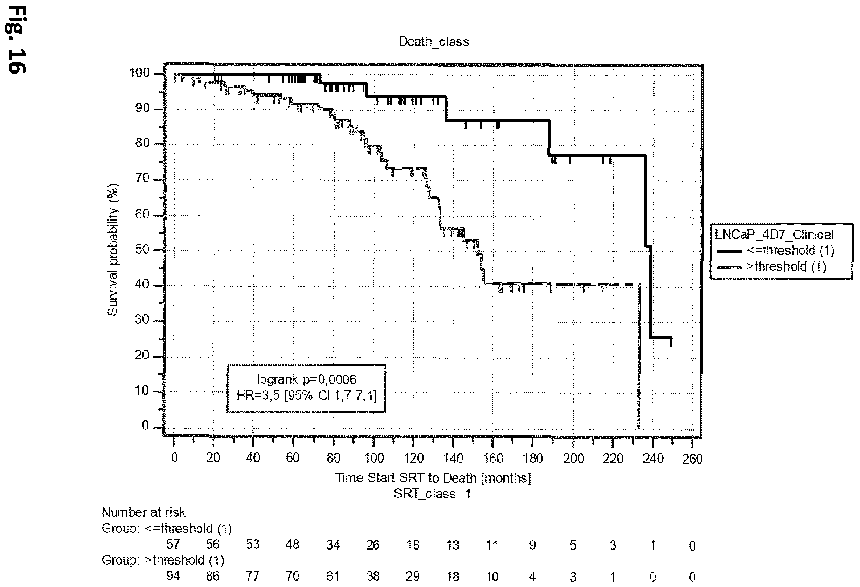

Fig. 16 shows a Kaplan-Meier curve of the PDE4D7_clinical_model in a 151 patient cohort (testing set used to validate the PDE4D7_clinical_model as developed on the training set) with all patients undergoing SRT (salvage radiation treatment) after post-surgical BCR (biochemical recurrence). The clinical endpoint that was tested was overall survival (Death) after the start of salvage radiation therapy (SRT) due to post-surgical disease recurrence. Logrank, HR and confidence interval are indicated in the figure. The included supplementary lists indicate the number of patients at risk for the PDE4D7_clinical_model classes analyzed, i.e., the patients at risk at any time interval +20 months after surgery are shown.

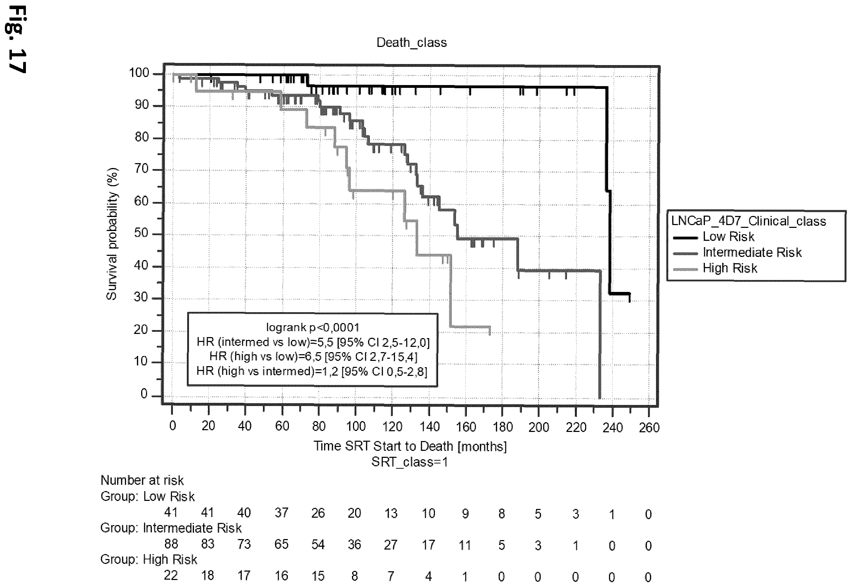

Fig. 17 shows a Kaplan-Meier curve of the PDE4D7_clinical class_model in a 151 patient cohort (testing set used to validate the PDE4D7_clinical class_model as developed on the training set) with all patients undergoing SRT (salvage radiation treatment) after post-surgical BCR (biochemical recurrence). The clinical endpoint that was tested was overall survival (Death) after the start of salvage radiation therapy (SRT) due to post-surgical disease recurrence. Logrank, HR and confidence interval are indicated in the figure. The included supplementary lists indicate the number of patients at risk for the PDE4D7_clinical class_model classes analyzed, i.e., the patients at risk at any time interval +20 months after surgery are shown.

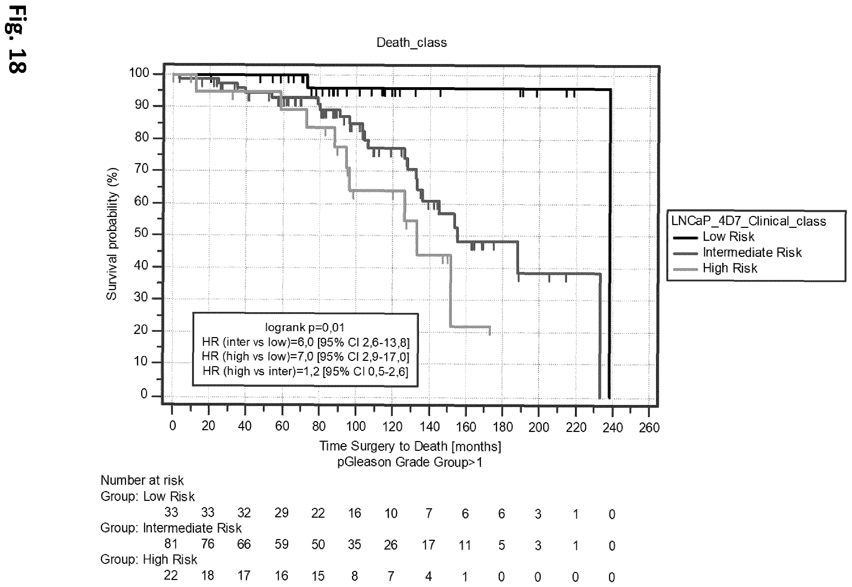

Fig. 18 shows a Kaplan-Meier curve of the PDE4D7_clinical class_model in a 136 patient cohort (testing set used to validate the PDE4D7_clinical class_model as developed on the training set) with all patients undergoing SRT (salvage radiation treatment) after post-surgical BCR (biochemical recurrence). The clinical endpoint that was tested was overall survival (Death) after the start of salvage radiation therapy (SRT) due to post-surgical disease recurrence. Logrank, HR and confidence interval are indicated in the figure. The included supplementary lists indicate the number of patients at risk for the PDE4D7_clinical class_model classes analyzed, i.e., the patients at risk at any time interval +20 months after surgery are shown.

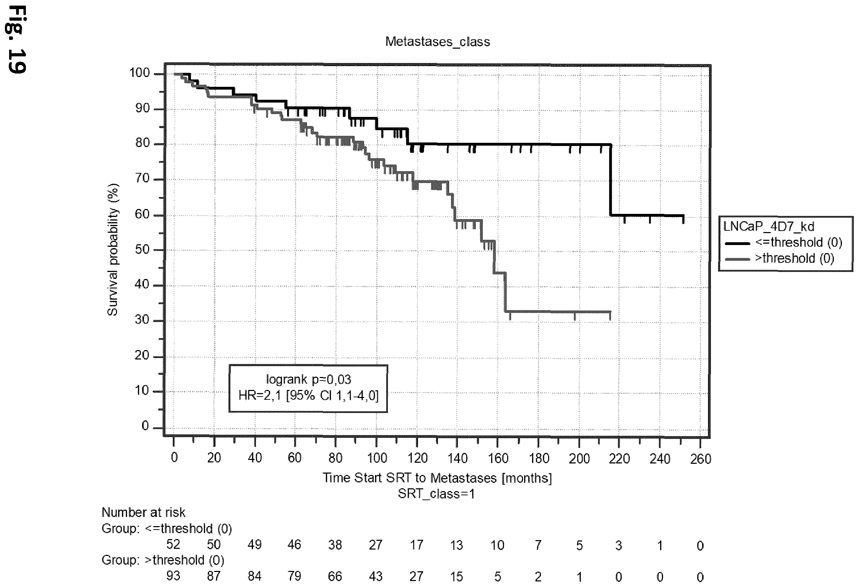

Fig. 19 shows a Kaplan-Meier curve of the PDE4D7_KD_model in a 145 patient cohort (testing set used to validate the PDE4D7_KD_model as developed on the training set) with all patients undergoing SRT (salvage radiation treatment) after post-surgical BCR (biochemical recurrence). The clinical endpoint that was tested was metastases progression free survival (metastasis) after the start of salvage radiation therapy (SRT) due to post-surgical disease recurrence. Logrank, HR and confidence interval are indicated in the figure. The included supplementary lists indicate the number of patients at risk for the PDE4D7_KD_model classes analyzed, i.e., the patients at risk at any time interval +20 months after surgery are shown.

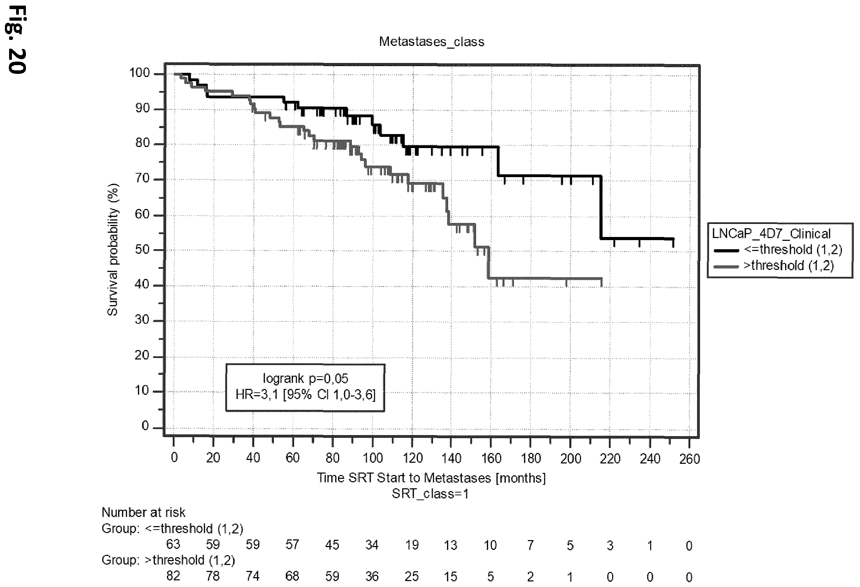

Fig. 20 shows a Kaplan-Meier curve of the PDE4D7_clinical_model in a 145 patient cohort (testing set used to validate the PDE4D7_clinical_model) with all patients undergoing SRT (salvage radiation treatment) after post-surgical BCR (biochemical recurrence). The clinical endpoint that was tested was metastases progression free survival (metastasis) after the start of salvage radiation therapy (SRT) due to post-surgical disease recurrence. Logrank, HR and confidence interval are indicated in the figure. The included supplementary lists indicate the number of patients at risk for the PDE4D7_clinical_model classes analyzed, i.e., the patients at risk at any time interval +20 months after surgery are shown.

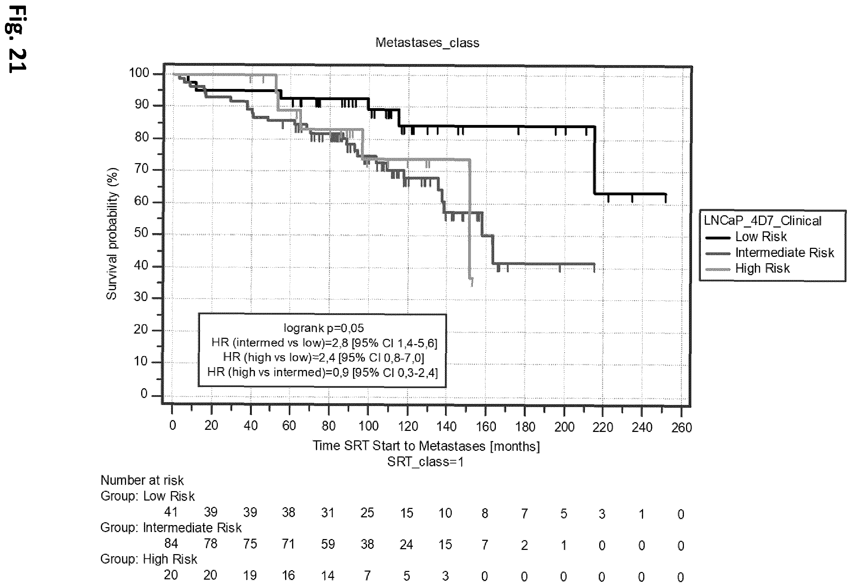

Fig. 21 shows a Kaplan-Meier curve of the PDE4D7_clinical_model in a 145 patient cohort (testing set used to validate the PDE4D7_clinical_model) with all patients undergoing SRT (salvage radiation treatment) after post-surgical BCR (biochemical recurrence). The clinical endpoint that was tested was metastases progression free survival (metastasis) after the start of salvage radiation therapy (SRT) due to post-surgical disease recurrence. Logrank, HR and confidence interval are indicated in the figure. The included supplementary lists indicate the number of patients at risk for the PDE4D7_clinical_model classes analyzed, i.e., the patients at risk at any time interval +20 months after surgery are shown.

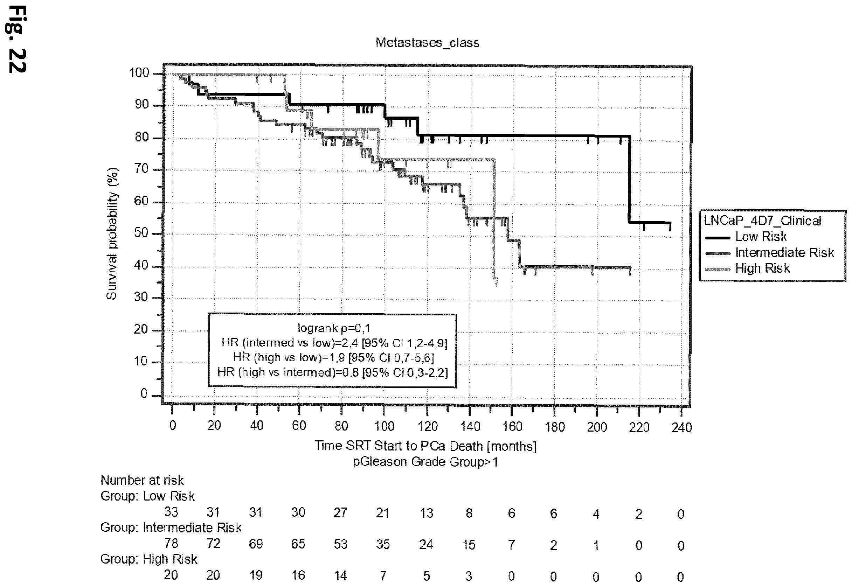

Fig. 22 shows a Kaplan-Meier curve of the PDE4D7_clinical_model in a 131 patient cohort (testing set used to validate the PDE4D7_clinical_model) with all patients undergoing SRT (salvage radiation treatment) after post-surgical BCR (biochemical recurrence). The clinical endpoint that was tested was metastases progression free survival (metastasis) after the start of salvage radiation therapy (SRT) due to post-surgical disease recurrence. Logrank, HR and confidence interval are indicated in the figure. The included supplementary lists indicate the number of patients at risk for the PDE4D7_clinical_model classes analyzed, i.e., the patients at risk at any time interval +20 months after surgery are shown.

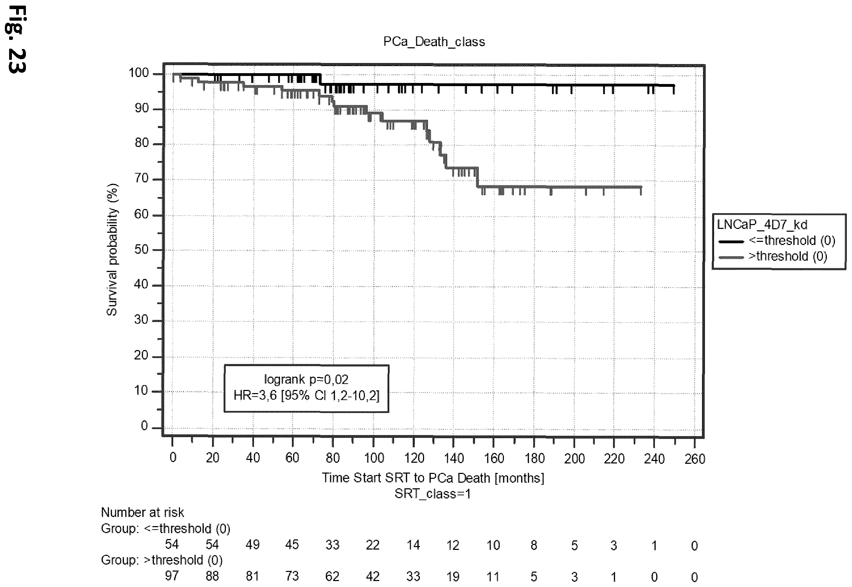

Fig. 23 shows a Kaplan-Meier curve of the PDE4D7_KD_model in a 151 patient cohort (testing set used to validate the PDE4D7_KD_model) with all patients undergoing SRT (salvage radiation treatment) after post-surgical BCR (biochemical recurrence). The clinical endpoint that was tested was prostate cancer specific survival (PCa Death) after the start of salvage radiation therapy (SRT) due to post-surgical disease recurrence. Logrank, HR and confidence interval are indicated in the figure. The included supplementary lists indicate the number of patients at risk for the PDE4D7_KD_model classes analyzed, i.e., the patients at risk at any time interval +20 months after surgery are shown.

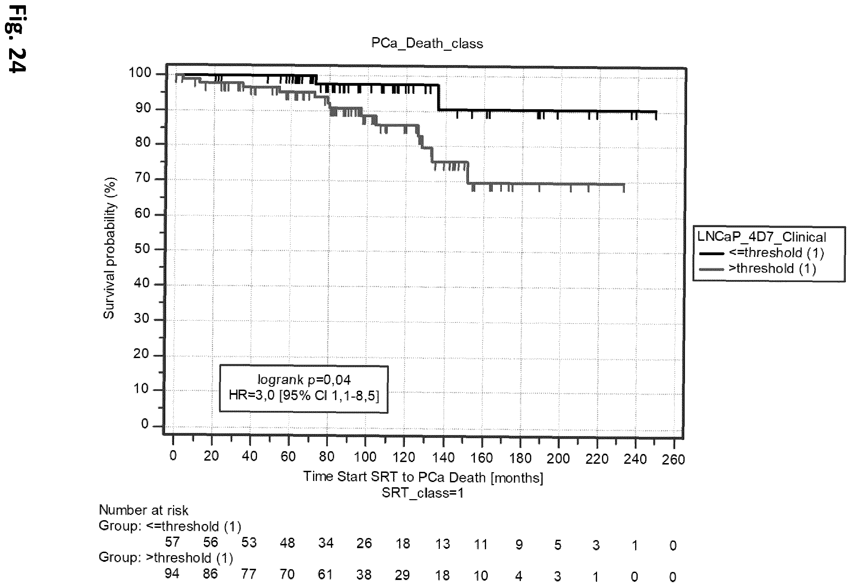

Fig. 24 shows a Kaplan-Meier curve of the PDE4D7_clinical_model in a 151 patient cohort (testing set used to validate the PDE4D7_clinical_model) with all patients undergoing SRT (salvage radiation treatment) after post-surgical BCR (biochemical recurrence). The clinical endpoint that was tested was prostate cancer specific survival (PCa Death) after the start of salvage radiation therapy (SRT) due to post-surgical disease recurrence. Logrank, HR and confidence interval are indicated in the figure. The included supplementary lists indicate the number of patients at risk for the PDE4D7_clinical_model classes analyzed, i.e., the patients at risk at any time interval +20 months after surgery are shown.

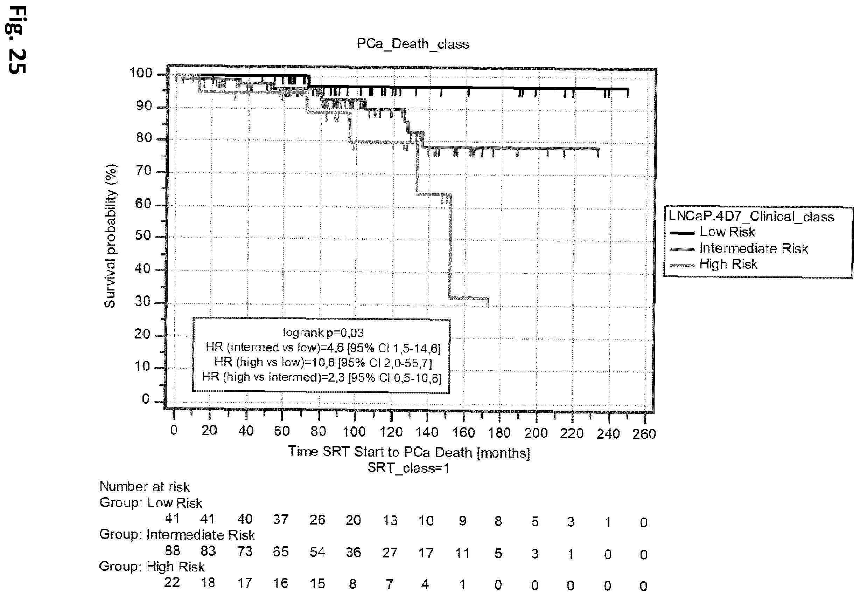

Fig. 25 shows a Kaplan-Meier curve of the PDE4D7_clinical class_model in a 151 patient cohort (testing set used to validate the PDE4D7_clinical class model) with all patients undergoing SRT (salvage radiation treatment) after post-surgical BCR (biochemical recurrence). The clinical endpoint that was tested was prostate cancer specific survival (PCa Death) after the start of salvage radiation therapy (SRT) due to post-surgical disease recurrence. Logrank, HR and confidence interval are indicated in the figure. The included supplementary lists indicate the number of patients at risk for the PDE4D7_clinical class_model classes analyzed, i.e., the patients at risk at any time interval +20 months after surgery are shown.

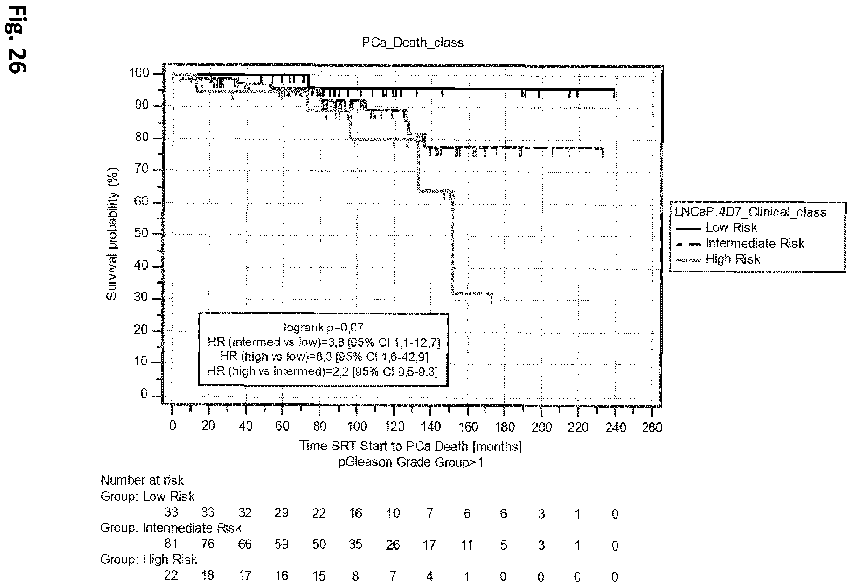

Fig. 26 shows a Kaplan-Meier curve of the PDE4D7_clinical class_model in a 136 patient cohort (testing set used to validate the PDE4D7_clinical class model) with all patients undergoing SRT (salvage radiation treatment) after post-surgical BCR (biochemical recurrence). The clinical endpoint that was tested was prostate cancer specific survival (PCa Death) after the start of salvage radiation therapy (SRT) due to post-surgical disease recurrence. Logrank, HR and confidence interval are indicated in the figure. The included supplementary lists indicate the number of patients at risk for the PDE4D7_clinical class_model classes analyzed, i.e., the patients at risk at any time interval +20 months after surgery are shown.

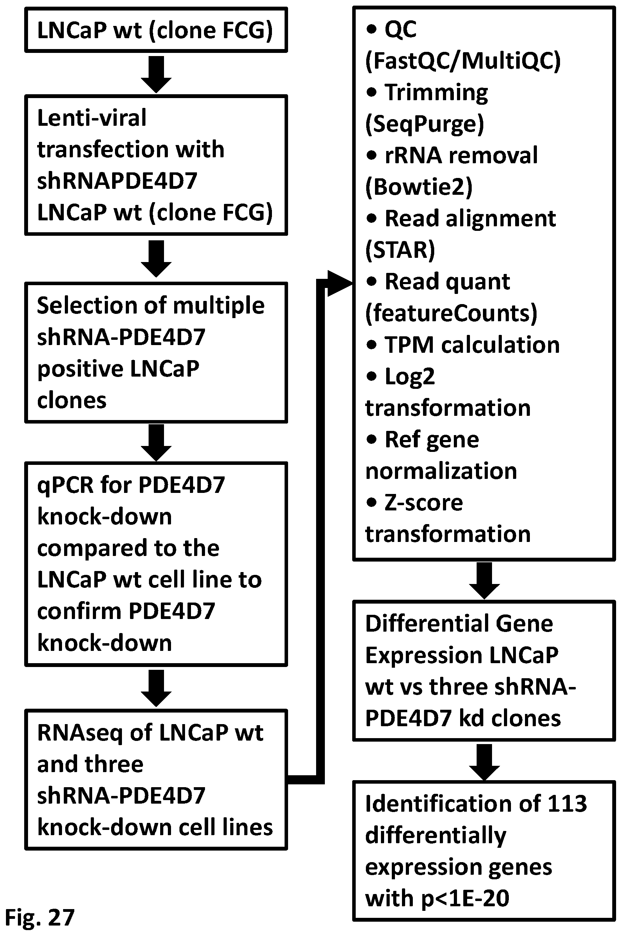

Fig. 27 depicts a schematic overview of the know-down strategy used to identify the broad set PDE4D7 knock-down genes (113 genes).

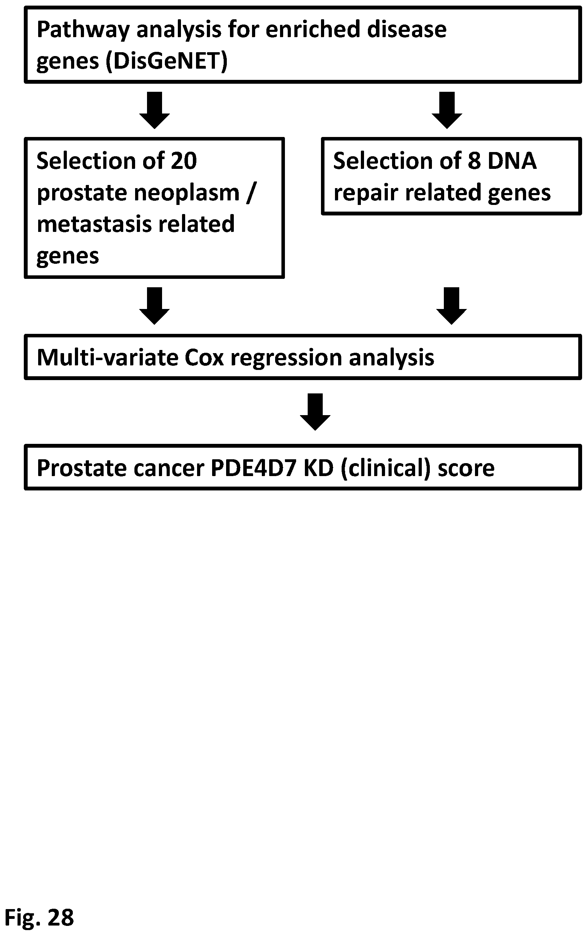

Fig. 28 depicts a schematic overview of the strategy used to select the 28 gene set and validation thereof.

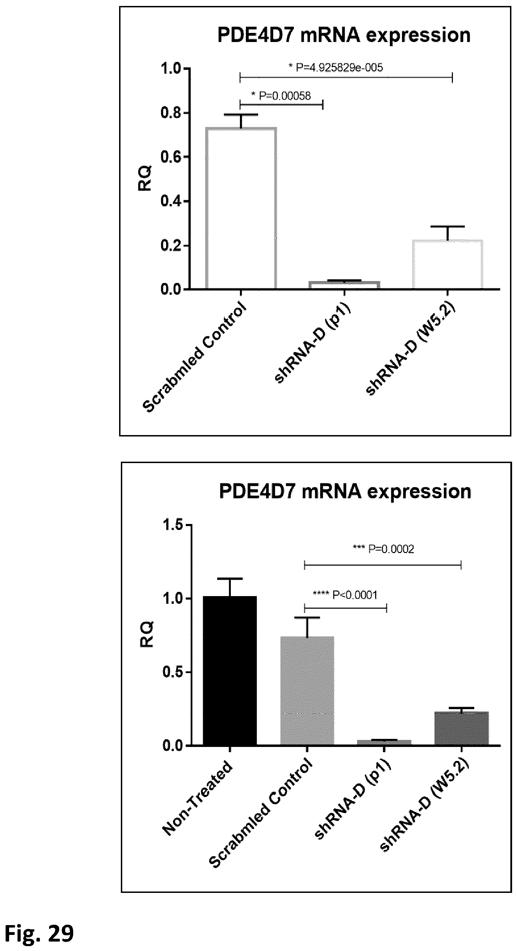

Fig. 29 depicts PDE4D7 mRNA expression levels in control (scambled (top); non-treated and scrambled (bottom)) in LNCaP cells and PDE4D7 shRNA transfected LNCaP cells.

## DETAILED DESCRIPTION OF THE EMBODIMENTS

Phosphodiesterases (PDEs) provide the sole means for the degradation of the second messenger 3'-5'-cyclic AMP. As such they are poised to provide a key regulatory role. Thus, aberrant changes in their expression, activity and intracellular location may all contribute to the underlying molecular pathology of particular disease states. Indeed, it has recently been shown that mutations in PDE genes are enriched in prostate cancer patients leading to elevated cAMP signalling and a potential predisposition to prostate cancer. However, varied expression profiles in different cell types coupled with complex arrays of isoform variants within each PDE family makes understanding the links between aberrant changes in PDE expression and functionality during disease progression challenging. Several studies have endeavored to describe the complement of PDEs in prostate, all of which identified significant levels of PDE4 expression alongside other PDEs.

Using sequence information on currently identified PDE isoforms, we have previously analysed their expression in 19 prostate cancer cell lines and xenografts [Henderson, 2014]. Such studies identified PDE3B, PDE4B, PDE4D, PDE7A, PDE8A, PDE8B and PDE9A isoforms as being abundantly expressed at the mRNA level in cancerous prostate cells [Henderson, 2014], while PDE1, PDE3A, PDE5A, PDE10A and PDE11A mRNA are present at lower levels (unpublished data), highlighting the complexity of cyclic nucleotide signalling in the prostate epithelium. Importantly, by separating the prostate cancer cell samples into androgen sensitive and androgen insensitive, castration resistant prostate cancer (CRPC), cellular phenotypes, we discovered that the expression of PDE4D isoforms was down-regulated in CRPC samples. In particular, we found that the most abundant PDE4 isoform in many of the androgen sensitive samples, PDE4D7, exhibited a significant degree of down-regulation in the CRPC cell models, presenting a scenario where the down-regulation of PDE4D7 could directly contribute to the exacerbation of disease driving cAMP signalling changes. Moreover, these observations suggested that measurement of PDE4D7 may inform on prostate cancer disease progression where low levels of expression may be connected with a more aggressive phenotype.

Based on the correlation between PDE4D7 expression and pathological features of the disease, our defined aim was to identify prognostic associations between the expression of PDE4D7 in a patient prostate tissue, collected by either biopsy or surgery, and clinically useful information relevant to the outcome of individual patients. Clinically relevant endpoints, or surrogate endpoints that are significantly correlated to the development of metastases, cancer specific or overall mortality have, typically, been evaluated as prognostic cancer biomarkers. The most relevant rational for using a surrogate endpoint relates to situations where either data on established clinical endpoints are not available or when the number of events in the data cohort is too limited for statisctical data analysis. For the development of the PDE4D7 prognostic biomarker we evaluated either BCR (biochemical relapse) progression-free survival or start of post-surgical secondary treatment as surrogate endpoints for metastases and prostate cancer death. Using these particular endpoints we identified a relevant number of events in our clinical cohorts (e.g., >30% for BCR), which is particularly relevant for multivariable data analysis.

In our evaluation, we selected standard methods of multivariable analysis such as Cox regression and Kaplan-Meier survival analysis in order to investigate the added and independent value of the continuous and/or the categorical 'PDE4D7 score' compared to established prognostic clinical variables such as PSA and Gleason score [Alves de Inda, 2018]. We thus built risk models where we combined the 'PDE4D7 score' with either pre- or post-surgical clinical predictors of post-surgical progression using logistic regression. The resulting models were subsequently tested on multiple independent patient cohorts in Kaplan-Meier survival and ROC curve analysis in order to predict post-treatment progression free survival [Alves de Inda, 2018].

Using such a strategy, we set out to test the prognostic value of the PDE4D7 score on a biopsy from retrospectively collected, resected prostate tissue in a consecutively managed patient cohort from a single surgery center in a post-surgical setting [Alves de Inda, 2018]. The patient population comprised some 500 individuals where longitudinal follow-up, of both pathology and biological outcomes, was undertaken. These clinical data were available for all patients and collected during a follow-up of a median 120 months after treatment. The 'PDE4D7 score' was determined as described above and then tested in both uni- and multivariable analyses using the available post-surgical co-variates (i.e. pathology Gleason score, pT stage, surgical margin status, seminal vesicle invasion status, and lymph node invasion status) in order to adjust for the multivariable setting. In this instance, biochemical progression-free survival after primary intervention was set as the evaluated clinical endpoint. The univariable analysis of these clinical samples [Alves de Inda, 2018], showing the inverse association between PDE4D7 expression (in terms of 'PDE4D7 score') and post-surgical biological relapse (HR=0.53 per unit change; 95% CI 0.41-0.67; p<0.0001), robustly confirmed our previous data [Boettcher 2015; Boettcher, 2016]. In multivariable analysis with such clinical variables, the 'PDE4D7 score' remained as an independent and effective means for predicting clinical outcome (HR=0.56 per unit change; 95% CI 0.43-0.73; p<0.0001). Furthermore, we obtained a very similar outcome when we evaluated the 'PDE4D7 score' in multivariable analysis (HR=0.54 95% CI 0.42-0.69; p<0.0001) with the validated and clinically-used risk model CAPRA-S. The CAPRA-S score, which is based on pre-operative PSA and pathologic parameters determined at the time of surgery, was developed to provide clinicians with information aimed to help predict disease recurrence, including BCR, systemic progression, and PCSM and has been validated in US and other populations.

Interestingly, when assessing the hazard ratio (HR) compared to the continuous 'PDE4D7 score' we uncovered a linear increase in risk with decreasing 'PDE4D7 score' for score values lying between 2 and 5. However, at PDE4D7 scores less than 2, then the risk of post-surgical progression increases steeply [Alves de Inda, 2018]. This is also evident in the Kaplan-Meier survival curves where patients that are grouped within the lowest 'PDE4D7 scores' category exhibit the highest risk of disease recurrence. Using logistic regression analysis we then combined the CAPRA-S score with the continuous 'PDE4D7 score'. Testing this model using ROC curve analysis we noticed a 4-6% significant improvement in AUC compared to the CAPRA-S alone for both 2- and 5-year predictions of post-treatment progression to BCR. Thus we evaluated a combined CAPRA-S & 'PDE4D7 score' Cox regression combination model in Kaplan-Meier survival analysis and compared this to the CAPRA-S score categories alone. Undertaking this, we confirmed the added value in risk prediction when using a model the combined 'PDE4D7 & CAPRA-S' score, compared to using the clinical metric of CAPRA-S score alone [Alves de Inda, 2018].

Subsequent to the diagnosis of prostate cancer, an accurate risk assessment needs to be undertaken before stratification to a defined primary treatment. With this in mind, we set out to see if we could translate the prognostic use of the 'PDE4D7 score' in a pre-surgery situation testing tumour tissue obtained from diagnostic needle biopsy samples [van Strijp 2018]. In this, needle biopsies were performed on 168 patients, from a single diagnostic clinical centre, who had undergone surgery as a primary treatment. The minimum follow-up period for each patient was 60 months after this intervention. The clinical co-variates used to adjust the 'PDE4D7 score' in the multivariable analysis were age at surgery, pre-operative PSA, PSA density, biopsy Gleason score, percentage of tumor positive biopsy cores, percentage of tumour in the biopsy and clinical cT stage. In this we evaluated the utility of the 'PDE4D7 score' and the combined 'PDE4D7 & CAPRA' scores compared to the pre-surgical CAPRA score in Cox regression analysis for biochemical relapse [van Strijp 2018].

Evaluating this patient cohort we found [van Strijp 2018] that the 'PDE4D7 score' was inversely associated with BCR in multivariable analysis when adjusting for clinical variables (HR=0.43; 95% CI 0.29-0.63; p<0.0001) as well as for the clinical CAPRA score (HR=0.53; 95% CI 0.38-0.74; p=0.0001). Kaplan-Meier analysis demonstrated that, as before, in a post-surgical setting, the 'PDE4D7 score' categories were significantly associated with BCR progression free survival (logrank p<0.0001) and secondary treatment free survival (logrank p=0.01). We then employed [van Strijp 2018] a combination logistic regression model, which was developed on the previous cohort. This consisted of the combined 'CAPRA & PDE4D7' score, demonstrating that patients within the highest combined 'CAPRA & PDE4D7' combined score category have virtually no risk of biochemical progression or transfer to any secondary treatment after surgery. This logistic regression model was also evaluated using ROC curve analysis in order to predict 5-year BCR after surgery. This revealed an increase in AUC of 5% over the CAPRA score alone (AUC=0.82 vs. 0.77, respectively; p=0.004). Decision curve analysis of the combined 'CAPRA & PDE4D7' score model confirmed the superior net benefit of using this combined score, compared to either score alone, across all decision thresholds in order to decide on whether to undertake intervention (e.g. surgery) based on the risk threshold of an individual patient to experience post-surgical disease progression [van Strijp 2018].

The effectiveness of both radical RT and SRT for localized prostate cancer is limited, resulting in disease progression and ultimately death of patients, especially for those at high risk of recurrence. The prediction of the therapy outcome is very complicated as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the response to radical RT and to SRT, in order to increase the success rate of these therapies.

We have here newly identified molecules of which expression shows a significant relation to mortality after radical RT and SRT and therefore are expected to improve the prediction of the effectiveness of these treatments, by using a PDE4D7 knockdown strategy. Using the LNCaP prostate cancer cell line stable PDE4D7 knockdown lines were generated and analyzed. Using this method genes differentially expressed in the knockdown cell lines were tested and validated in human patient cohorts for their predictive value for predicting a response to radiotherapy. It was found that the genes ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2 each individually or when combined are able to predict favorable or poor response in a prostate cancer subject to radiotherapy.

Therefore, in a first aspect, the invention relates to a method of predicting a response of a prostate cancer subject to radiotherapy, comprising:

- determining or receiving the result of a determination of the gene expression levels for each of three or more genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, said gene expression levels being determined in a biological sample obtained from the subject,
- determining the prediction of the radiotherapy response based on the gene expression levels for the three or more genes, wherein said prediction is a favorable repsonse or a non-favorable response to radiotherapy.

Using sequence information on currently identified PDE isoforms, we have analysed their expression in 19 prostate cancer cell lines and xenografts (see Henderson D.J. et al., "The cAMP phosphodiesterase-4D7 (PDE4D7) is downregulated in androgen-independent prostate cancer cells and mediates proliferation by compartmentalizing cAMP at the plasma membrane of VCaP prostate cancer cells", Br J Cancer, Vol. 110, No. 5, pages 1278-1287, 2014). Such studies identified PDE3B, PDE4B, PDE4D, PDE7A, PDE8A, PDE8B and PDE9A isoforms as being abundantly expressed at the mRNA level in cancerous prostate cells (see Henderson D.J. et al., 2014, ibid), while PDE1, PDE3A, PDE5A, PDE10A and PDE11A mRNA are present at lower levels (unpublished data), highlighting the complexity of cyclic nucleotide signalling in the prostate epithelium. Importantly, by separating the prostate cancer cell samples into androgen sensitive and androgen insensitive, castration resistant prostate cancer (CRPC), cellular phenotypes, we discovered that the expression of PDE4D isoforms was down-regulated in CRPC samples. In particular, we found that the most abundant PDE4 isoform in many of the androgen sensitive samples, PDE4D7, exhibited a significant degree of down-regulation in the CRPC cell models, presenting a scenario where the down-regulation of PDE4D7 could directly contribute to the exacerbation of disease driving cAMP signalling changes. Moreover, these observations suggested that measurement of PDE4D7 may inform on prostate cancer disease progression where low levels of expression may be connected with a more aggressive phenotype.

Based on the correlation between PDE4D7 expression and pathological features of the disease, our defined aim was to identify prognostic associations between the expression of PDE4D7 in a patient prostate tissue, collected by either biopsy or surgery, and clinically useful information relevant to the outcome of individual patients. Clinically relevant endpoints, or surrogate endpoints that are significantly correlated to the development of metastases, cancer specific or overall mortality have, typically, been evaluated as prognostic cancer biomarkers. The most relevant rational for using a surrogate endpoint relates to situations where either data on established clinical endpoints are not available or when the number of events in the data cohort is too limited for statistical data analysis. For the development of the PDE4D7 prognostic biomarker we evaluated either BCR (biochemical relapse) progression-free survival or start of post-surgical secondary treatment as surrogate endpoints for metastases and prostate cancer death. Using these particular endpoints we identified a relevant number of events in our clinical cohorts (e.g., >30% for BCR), which is particularly relevant for multivariable data analysis.

In our evaluation, we selected standard methods of multivariable analysis such as Cox regression and Kaplan-Meier survival analysis in order to investigate the added and independent value of the continuous and/or the categorical 'KD score' compared to established prognostic clinical variables such as PSA and Gleason score (see Alves de Inda M. et al., "Validation of Cyclic Adenosine Monophosphate Phosphodiesterase-4D7 for its Independent Contribution to Risk Stratification in a Prostate Cancer Patient Cohort with Longitudinal Biological Outcomes", Eur Urol Focus, Vol. 4, No. 3, pages 376-384, 2018). We thus built risk models where we combined the 'KD score' with either pre- or post-surgical clinical predictors of post-surgical progression using logistic regression. The resulting models were subsequently tested on multiple independent patient cohorts in Kaplan-Meier survival and ROC curve analysis in order to predict post-treatment progression free survival (see Alves de Inda M. et al., 2018, ibid).

Using such a strategy, we set out to test the prognostic value of the KD score on a biopsy from retrospectively collected, resected prostate tissue in a consecutively managed patient cohort from a single surgery center in a post-surgical setting (see Alves de Inda M. et al., 2018, ibid). The patient population comprised some 500 individuals where longitudinal follow-up, of both pathology and biological outcomes, was undertaken. These clinical data were available for all patients and collected during a follow-up of a median 120 months after treatment. The 'KD score' was determined as described above and then tested in both uni- and multivariable analyses using the available post-surgical co-variates (i.e. pathology Gleason score, pT stage, surgical margin status, seminal vesicle invasion status, and lymph node invasion status) in order to adjust for the multivariable setting. In this instance, biochemical progression-free survival after primary intervention was set as the evaluated clinical endpoint.

The present invention is based on the idea that, since the PDE4D7 biomarker has been proven to be a good predictor of radiotherapy response, the ability to identify markers that are differentially expressed upon PDE4D7 knock-down might help to be better able to predict overall RT response. To this extent, the strategy was followed as described in the examples to identify a group of 28 geness (ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2) which can be used individually or together to predict a response of a prostate cancer subject to radiotherapy.

The term "ACPP" refers to acid phosphatase 3 gene and is also known as ACP3 (Ensembl: ENSG00000014257; HGNC: 125). For example, an exemplary splice variant of the gene is set forth in the nucleotide sequence as defined in NCBI Reference Sequence NM_001099.5 which encodes the coding sequence CCDS3073 or the nucleotide sequence as set forth in SEQ ID NO: 1, which correspond to the sequence of the above indicated coding sequence of the ACPP transcript, and and encodes the corresponding amino acid sequence for example as set forth in SEQ ID NO:2, which correspond to the protein sequences defined in NCBI Protein Accession Reference Sequence NP_001090.2 encoding the ACPP polypeptide.

The term "ACPP" also comprises nucleotide sequences showing a high degree of homology to ACPP, e.g., nucleic acid sequences being at least 75%, 80%, 85%, 90%, 91%, 92%, 93%, 94%, 95%, 96%, 97%, 98% or 99% identical to the sequence as set forth in SEQ ID NO:1 or amino acid sequences being at least 75%, 80%, 85%, 90%, 91%, 92%, 93%, 94%, 95%, 96%, 97%, 98% or 99% identical to the sequence as set forth in SEQ ID NO:2 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:2 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:1.

The term "AR" refers to the androgen receptor gene (Ensembl: ENSG00000169083; HGNC: 644). For example, an exemplary splice variant of the gene is set forth in the nucleotide sequence as defined in NCBI Reference Sequence NM_000044.6 which encodes the coding sequence CCDS87754 or the nucleotide sequence as set forth in SEQ ID NO:3, which correspond to the sequence of the above indicated coding sequence of the AR transcript, and and encodes the corresponding amino acid sequence for example as set forth in SEQ ID NO:4, which correspond to the protein sequences defined in NCBI Protein Accession Reference Sequence NP_000035.2encoding the AR polypeptide.

The term "AR" also comprises nucleotide sequences showing a high degree of homology to AR, e.g., nucleic acid sequences being at least 75%, 80%, 85%, 90%, 91%, 92%, 93%, 94%, 95%, 96%, 97%, 98% or 99% identical to the sequence as set forth in SEQ ID NO:3 or amino acid sequences being at least 75%, 80%, 85%, 90%, 91%, 92%, 93%, 94%, 95%, 96%, 97%, 98% or 99% identical to the sequence as set forth in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:3.

The term "CDH1" refers to the cadherin 1 gene (Ensembl: ENSG00000039068; HGNC: 1748). For example, an exemplary splice variant of the gene is set forth in the nucleotide sequence as defined in NCBI Reference Sequence NM_004360.5 which encodes the coding sequence CCDS82005 or the nucleotide sequence as set forth in SEQ ID NO:5, which correspond to the sequence of the above indicated coding sequence of the CDH1 transcript, and and encodes the corresponding amino acid sequence for example as set forth in SEQ ID NO:6, which correspond to the protein sequences defined in NCBI Protein Accession Reference Sequence NP_004351.1 encoding the CDH1 polypeptide.

The term "CDH1" also comprises nucleotide sequences showing a high degree of homology to CDH1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:5.

The term "EHF" refers to the ETS homologous factor gene (Ensembl: ENSG00000135373; HGNC: 3246). For example, an exemplary splice variant of the gene is set forth in the nucleotide sequence as defined in NCBI Reference Sequence NM_012153.6 which encodes the coding sequence CCDS55752 or the nucleotide sequence as set forth in SEQ ID NO:7, which correspond to the sequence of the above indicated coding sequence of the EHF transcript, and and encodes the corresponding amino acid sequence for example as set forth in SEQ ID NO:8, which correspond to the protein sequences defined in NCBI Protein Accession Reference Sequence NP_036285.2 encoding the EHF polypeptide.

The term "EHF" also comprises nucleotide sequences showing a high degree of homology to EHF, e.g., nucleic acid sequences being at least 75%, 80%, 85%, 90%, 91%, 92%, 93%, 94%, 95%, 96%, 97%, 98% or 99% identical to the sequence as set forth in SEQ ID NO:7 or amino acid sequences being at least 75%, 80%, 85%, 90%, 91%, 92%, 93%, 94%, 95%, 96%, 97%, 98% or 99% identical to the sequence as set forth in SEQ ID NO:8 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:8 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:7.

The term "ETV1" refers to the ETS variant 1 gene (Ensembl: ENSG00000006468; HGNC: 3490). For example, an exemplary splice variant of the gene is set forth in the nucleotide sequence as defined in NCBI Reference Sequence NM_004956.5 which encodes the coding sequence CCDS55085 or the nucleotide sequence as set forth in SEQ ID NO:9, which correspond to the sequence of the above indicated coding sequence of the ETV1 transcript, and and encodes the corresponding amino acid sequence for example as set forth in SEQ ID NO:10, which correspond to the protein sequences defined in NCBI Protein Accession Reference Sequence NP_004947.2 encoding the ETV1 polypeptide.

The term "ETV1" also comprises nucleotide sequences showing a high degree of homology to ETV1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:9 or amino acid sequences being at least 75%, 80%, 85%, 90%, 91%, 92%, 93%, 94%, 95%, 96%, 97%, 98% or 99% identical to the sequence as set forth in SEQ ID NO:10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:9.

The term "FOLH1" refers to the folate hydrolase 1 gene (Ensembl: ENSG00000086205; HGNC: 3788). For example, an exemplary splice variant of the gene is set forth in the nucleotide sequence as defined in NCBI Reference Sequence NM_004476.3 which encodes the coding sequence CCDS31493 or the nucleotide sequence as set forth in SEQ ID NO:11, which correspond to the sequence of the above indicated coding sequence of the FOLH1 transcript, and and encodes the corresponding amino acid sequence for example as set forth in SEQ ID NO:12, which correspond to the protein sequences defined in NCBI Protein Accession Reference Sequence NP_004467.1 encoding the FOLH1 polypeptide.

The term "FOLH1" also comprises nucleotide sequences showing a high degree of homology to FOLH1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:11 or amino acid sequences being at least 75%, 80%, 85%, 90%, 91%, 92%, 93%, 94%, 95%, 96%, 97%, 98% or 99% identical to the sequence as set forth in SEQ ID NO:12 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:12 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:11.

The term "FOXA1" refers to the forkhead box A1 gene (Ensembl: ENSG00000129514; HGNC: 5021). For example, an exemplary splice variant of the gene is set forth in the nucleotide sequence as defined in NCBI Reference Sequence NM_004496.5 which encodes the coding sequence CCDS9665 or the nucleotide sequence as set forth in SEQ ID NO:13, which correspond to the sequence of the above indicated coding sequence of the FOXA1 transcript, and and encodes the corresponding amino acid sequence for example as set forth in SEQ ID NO:14, which correspond to the protein sequences defined in NCBI Protein Accession Reference Sequence NP_004487.2 encoding the FOXA1 polypeptide.

The term "FOXA1" also comprises nucleotide sequences showing a high degree of homology to FOXA1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:13 or amino acid sequences being at least 75%, 80%, 85%, 90%, 91%, 92%, 93%, 94%, 95%, 96%, 97%, 98% or 99% identical to the sequence as set forth in SEQ ID NO:14 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:14 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:13.

The term "HOXB13" refers to the homeobox B13gene (Ensembl: ENSG00000159184; HGNC: 5112). For example, an exemplary splice variant of the gene is set forth in the nucleotide sequence as defined in NCBI Reference Sequence NM_006361.6 which encodes the coding sequence CCDS11536 or the nucleotide sequence as set forth in SEQ ID NO:15, which correspond to the sequence of the above indicated coding sequence of the HOXB13 transcript, and and encodes the corresponding amino acid sequence for example as set forth in SEQ ID NO:16, which correspond to the protein sequences defined in NCBI Protein Accession Reference Sequence NP_006352.2 encoding the HOXB13 polypeptide.

The term "HOXB13" also comprises nucleotide sequences showing a high degree of homology to HOXB13, e.g., nucleic acid sequences being at least 75%, 80%, 85%, 90%, 91%, 92%, 93%, 94%, 95%, 96%, 97%, 98% or 99% identical to the sequence as set forth in SEQ ID NO:15 or amino acid sequences being at least 75%, 80%, 85%, 90%, 91%, 92%, 93%, 94%, 95%, 96%, 97%, 98% or 99% identical to the sequence as set forth in SEQ ID NO:16 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:16 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:15.

The term "KLK2" refers to the kallikrein related peptidase 2 gene (Ensembl: ENSG00000167751; HGNC: 6363). For example, an exemplary splice variant of the gene is set forth in the nucleotide sequence as defined in NCBI Reference Sequence NM_005551.5 which encodes the coding sequence CCDS12808 or the nucleotide sequence as set forth in SEQ ID NO:17, which correspond to the sequence of the above indicated coding sequence of the KLK2 transcript, and and encodes the corresponding amino acid sequence for example as set forth in SEQ ID NO:18, which correspond to the protein sequences defined in NCBI Protein Accession Reference Sequence NP_005542.1 encoding the KLK2 polypeptide.

The term "KLK2" also comprises nucleotide sequences showing a high degree of homology to KLK2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:17 or amino acid sequences being at least 75%, 80%, 85%, 90%, 91%, 92%, 93%, 94%, 95%, 96%, 97%, 98% or 99% identical to the sequence as set forth in SEQ ID NO:18 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:17.

The term "KLK3" refers to the kallikrein related peptidase 3 gene (Ensembl: ENSG00000142515; HGNC: 6364). For example, an exemplary splice variant of the gene is set forth in the nucleotide sequence as defined in NCBI Reference Sequence NM_001648.2 which encodes the coding sequence CCDS12807 or the nucleotide sequence as set forth in SEQ ID NO:19, which correspond to the sequence of the above indicated coding sequence of the KLK3 transcript, and and encodes the corresponding amino acid sequence for example as set forth in SEQ ID NO:20, which correspond to the protein sequences defined in NCBI Protein Accession Reference Sequence NP_001639.1 encoding the KLK3 polypeptide.

The term "KLK3" also comprises nucleotide sequences showing a high degree of homology to KLK3, e.g., nucleic acid sequences being at least 75%, 80%, 85%, 90%, 91%, 92%, 93%, 94%, 95%, 96%, 97%, 98% or 99% identical to the sequence as set forth in SEQ ID NO:19 or amino acid sequences being at least 75%, 80%, 85%, 90%, 91%, 92%, 93%, 94%, 95%, 96%, 97%, 98% or 99% identical to the sequence as set forth in SEQ ID NO:20 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:19.

The term "MAOA" refers to the monoamine oxidase A gene (Ensembl: ENSG00000189221; HGNC: 6833). For example, an exemplary splice variant of the gene is set forth in the nucleotide sequence as defined in NCBI Reference Sequence NM_000240.4 which encodes the coding sequence CCDS14260 or the nucleotide sequence as set forth in SEQ ID NO:21, which correspond to the sequence of the above indicated coding sequence of the MAOA transcript, and and encodes the corresponding amino acid sequence for example as set forth in SEQ ID NO:22, which correspond to the protein sequences defined in NCBI Protein Accession Reference Sequence NP_000231.1 encoding the MAOA polypeptide.

The term "MAOA" also comprises nucleotide sequences showing a high degree of homology to MAOA, e.g., nucleic acid sequences being at least 75%, 80%, 85%, 90%, 91%, 92%, 93%, 94%, 95%, 96%, 97%, 98% or 99% identical to the sequence as set forth in SEQ ID NO:21 or amino acid sequences being at least 75%, 80%, 85%, 90%, 91%, 92%, 93%, 94%, 95%, 96%, 97%, 98% or 99% identical to the sequence as set forth in SEQ ID NO:22 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:22 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:21.

The term "MLH1" refers to the mutL homolog 1 gene (Ensembl: ENSG00000076242; HGNC: 7127). For example, an exemplary splice variant of the gene is set forth in the nucleotide sequence as defined in NCBI Reference Sequence NM_000249.4 which encodes the coding sequence CCDS54562 or the nucleotide sequence as set forth in SEQ ID NO:23, which correspond to the sequence of the above indicated coding sequence of the MLH1 transcript, and and encodes the corresponding amino acid sequence for example as set forth in SEQ ID NO:24, which correspond to the protein sequences defined in NCBI Protein Accession Reference Sequence NP_000240.1 encoding the MLH1 polypeptide.

The term "MLH1" also comprises nucleotide sequences showing a high degree of homology to MLH1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:23 or amino acid sequences being at least 75%, 80%, 85%, 90%, 91%, 92%, 93%, 94%, 95%, 96%, 97%, 98% or 99% identical to the sequence as set forth in SEQ ID NO:24 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:24 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:23.

The term "MME" refers to the membrane metalloendopeptidase gene (Ensembl: ENSG00000196549; HGNC: 7154). For example, an exemplary splice variant of the gene is set forth in the nucleotide sequence as defined in NCBI Reference Sequence NM_007289.4 which encodes the coding sequence CCDS87157 or the nucleotide sequence as set forth in SEQ ID NO:25, which correspond to the sequence of the above indicated coding sequence of the MME transcript, and and encodes the corresponding amino acid sequence for example as set forth in SEQ ID NO:26, which correspond to the protein sequences defined in NCBI Protein Accession Reference Sequence NP_001341573.1 encoding the MME polypeptide.

The term "MME" also comprises nucleotide sequences showing a high degree of homology to MME, e.g., nucleic acid sequences being at least 75%, 80%, 85%, 90%, 91%, 92%, 93%, 94%, 95%, 96%, 97%, 98% or 99% identical to the sequence as set forth in SEQ ID NO:25 or amino acid sequences being at least 75%, 80%, 85%, 90%, 91%, 92%, 93%, 94%, 95%, 96%, 97%, 98% or 99% identical to the sequence as set forth in SEQ ID NO:26 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:26 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:25.

The term "MYO6" refers to the myosin VI gene (Ensembl: ENSG00000196586; HGNC: 7605). For example, an exemplary splice variant of the gene is set forth in the nucleotide sequence as defined in NCBI Reference Sequence NM_004999.4 which encodes the coding sequence CCDS34487 or the nucleotide sequence as set forth in SEQ ID NO:27, which correspond to the sequence of the above indicated coding sequence of the MYO6 transcript, and and encodes the corresponding amino acid sequence for example as set forth in SEQ ID NO:28, which correspond to the protein sequences defined in NCBI Protein Accession Reference Sequence NP_004990.3 encoding the MYO6 polypeptide.

The term "MYO6" also comprises nucleotide sequences showing a high degree of homology to MYO6, e.g., nucleic acid sequences being at least 75%, 80%, 85%, 90%, 91%, 92%, 93%, 94%, 95%, 96%, 97%, 98% or 99% identical to the sequence as set forth in SEQ ID NO:27 or amino acid sequences being at least 75%, 80%, 85%, 90%, 91%, 92%, 93%, 94%, 95%, 96%, 97%, 98% or 99% identical to the sequence as set forth in SEQ ID NO:28 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:28 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:27.

The term "NAALADL2" refers to the N-acetylated alpha-linked acidic dipeptidase like 2 gene (Ensembl: ENSG00000177694; HGNC: 23219). For example, an exemplary splice variant of the gene is set forth in the nucleotide sequence as defined in NCBI Reference Sequence NM_207015.3 which encodes the coding sequence CCDS46960 or the nucleotide sequence as set forth in SEQ ID NO:29, which correspond to the sequence of the above indicated coding sequence of the NAALADL2 transcript, and and encodes the corresponding amino acid sequence for example as set forth in SEQ ID NO:30, which correspond to the protein sequences defined in NCBI Protein Accession Reference Sequence NP_996898.2 encoding the NAALADL2 polypeptide.

The term "NAALADL2" also comprises nucleotide sequences showing a high degree of homology to NAALADL2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:29 or amino acid sequences being at least 75%, 80%, 85%, 90%, 91%, 92%, 93%, 94%, 95%, 96%, 97%, 98% or 99% identical to the sequence as set forth in SEQ ID NO:30 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:30 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:29.

The term "NKX3-1" refers to the NK3 homeobox 1 gene (Ensembl: ENSG00000167034; HGNC: 7838). For example, an exemplary splice variant of the gene is set forth in the nucleotide sequence as defined in NCBI Reference Sequence NM_006167.4 which encodes the coding sequence CCDS6042 or the nucleotide sequence as set forth in SEQ ID NO:31, which correspond to the sequence of the above indicated coding sequence of the NKX3-1 transcript, and and encodes the corresponding amino acid sequence for example as set forth in SEQ ID NO:32, which correspond to the protein sequences defined in NCBI Protein Accession Reference Sequence NP_006158.2 encoding the NKX3-1 polypeptide.

The term "NKX3-1" also comprises nucleotide sequences showing a high degree of homology to NKX3-1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:31 or amino acid sequences being at least 75%, 80%, 85%, 90%, 91%, 92%, 93%, 94%, 95%, 96%, 97%, 98% or 99% identical to the sequence as set forth in SEQ ID NO:32 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:32 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:31.

The term "NQO1" refers to the NAD(P)H quinone dehydrogenase 1 gene (Ensembl: ENSG00000181019; HGNC: 2874). For example, an exemplary splice variant of the gene is set forth in the nucleotide sequence as defined in NCBI Reference Sequence NM_000903.3 which encodes the coding sequence CCDS67067 or the nucleotide sequence as set forth in SEQ ID NO:33, which correspond to the sequence of the above indicated coding sequence of the NQO1 transcript, and and encodes the corresponding amino acid sequence for example as set forth in SEQ ID NO:34, which correspond to the protein sequences defined in NCBI Protein Accession Reference Sequence NP_001273066.1 encoding the NQO1 polypeptide.

The term "NQO1" also comprises nucleotide sequences showing a high degree of homology to NQO1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:33 or amino acid sequences being at least 75%, 80%, 85%, 90%, 91%, 92%, 93%, 94%, 95%, 96%, 97%, 98% or 99% identical to the sequence as set forth in SEQ ID NO:34 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:34 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:33.

The term "NRP1" refers to the neuropilin 1 gene (Ensembl: ENSG00000099250; HGNC: 8004). For example, an exemplary splice variant of the gene is set forth in the nucleotide sequence as defined in NCBI Reference Sequence NM_003873.7 which encodes the coding sequence CCDS7177 or the nucleotide sequence as set forth in SEQ ID NO:35, which correspond to the sequence of the above indicated coding sequence of the NRP1 transcript, and and encodes the corresponding amino acid sequence for example as set forth in SEQ ID NO:36, which correspond to the protein sequences defined in NCBI Protein Accession Reference Sequence NP_003864.5 encoding the NRP1 polypeptide.

The term "NRP1" also comprises nucleotide sequences showing a high degree of homology to NRP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:35 or amino acid sequences being at least 75%, 80%, 85%, 90%, 91%, 92%, 93%, 94%, 95%, 96%, 97%, 98% or 99% identical to the sequence as set forth in SEQ ID NO:36 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:36 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:35.

The term "SLC45A3" refers to the solute carrier family 45 member 3 gene (Ensembl: ENSG00000158715; HGNC: 8642). For example, an exemplary splice variant of the gene is set forth in the nucleotide sequence as defined in NCBI Reference Sequence NM_033102.3 which encodes the coding sequence CCDS1458 or the nucleotide sequence as set forth in SEQ ID NO:37, which correspond to the sequence of the above indicated coding sequence of the SLC45A3 transcript, and and encodes the corresponding amino acid sequence for example as set forth in SEQ ID NO:38, which correspond to the protein sequences defined in NCBI Protein Accession Reference Sequence NP_149093.1 encoding the SLC45A3 polypeptide.

The term "SLC45A3" also comprises nucleotide sequences showing a high degree of homology to SLC45A3, e.g., nucleic acid sequences being at least 75%, 80%, 85%, 90%, 91%, 92%, 93%, 94%, 95%, 96%, 97%, 98% or 99% identical to the sequence as set forth in SEQ ID NO:37 or amino acid sequences being at least 75%, 80%, 85%, 90%, 91%, 92%, 93%, 94%, 95%, 96%, 97%, 98% or 99% identical to the sequence as set forth in SEQ ID NO:38 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:38 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:37.

The term "SPDEF" refers to the SAM pointed domain containing ETS transcription factor gene (Ensembl: ENSG00000124664; HGNC: 17257). For example, an exemplary splice variant of the gene is set forth in the nucleotide sequence as defined in NCBI Reference Sequence NM_012391.3 which encodes the coding sequence CCDS4794 or the nucleotide sequence as set forth in SEQ ID NO:39, which correspond to the sequence of the above indicated coding sequence of the SPDEF transcript, and and encodes the corresponding amino acid sequence for example as set forth in SEQ ID NO:40, which correspond to the protein sequences defined in NCBI Protein Accession Reference Sequence NP_036523.1 encoding the SPDEF polypeptide.

The term "SPDEF" also comprises nucleotide sequences showing a high degree of homology to SPDEF, e.g., nucleic acid sequences being at least 75%, 80%, 85%, 90%, 91%, 92%, 93%, 94%, 95%, 96%, 97%, 98% or 99% identical to the sequence as set forth in SEQ ID NO:39 or amino acid sequences being at least 75%, 80%, 85%, 90%, 91%, 92%, 93%, 94%, 95%, 96%, 97%, 98% or 99% identical to the sequence as set forth in SEQ ID NO:40 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:40 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:39.

The term "ATM" refers to the ATM serine/threonine kinase gene (Ensembl: ENSG00000149311; HGNC: 795). For example, an exemplary splice variant of the gene is set forth in the nucleotide sequence as defined in NCBI Reference Sequence NM_000051.4 which encodes the coding sequence CCDS86245 or the nucleotide sequence as set forth in SEQ ID NO:41, which correspond to the sequence of the above indicated coding sequence of the ATM transcript, and and encodes the corresponding amino acid sequence for example as set forth in SEQ ID NO:42, which correspond to the protein sequences defined in NCBI Protein Accession Reference Sequence NP_000042.3 encoding the ATM polypeptide.

The term "ATM" also comprises nucleotide sequences showing a high degree of homology to ATM, e.g., nucleic acid sequences being at least 75%, 80%, 85%, 90%, 91%, 92%, 93%, 94%, 95%, 96%, 97%, 98% or 99% identical to the sequence as set forth in SEQ ID NO:41 or amino acid sequences being at least 75%, 80%, 85%, 90%, 91%, 92%, 93%, 94%, 95%, 96%, 97%, 98% or 99% identical to the sequence as set forth in SEQ ID NO:42 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:42 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:41.

The term "ATR" refers to the ATR serine/threonine kinase gene (Ensembl: ENSG00000175054; HGNC: 882). For example, an exemplary splice variant of the gene is set forth in the nucleotide sequence as defined in NCBI Reference Sequence NM_001184.4 which encodes the coding sequence CCDS3124 or the nucleotide sequence as set forth in SEQ ID NO:43, which correspond to the sequence of the above indicated coding sequence of the ATR transcript, and and encodes the corresponding amino acid sequence for example as set forth in SEQ ID NO:44, which correspond to the protein sequences defined in NCBI Protein Accession Reference Sequence NP_001175.2 encoding the ATR polypeptide.

The term "ATR" also comprises nucleotide sequences showing a high degree of homology to ATR, e.g., nucleic acid sequences being at least 75%, 80%, 85%, 90%, 91%, 92%, 93%, 94%, 95%, 96%, 97%, 98% or 99% identical to the sequence as set forth in SEQ ID NO:43 or amino acid sequences being at least 75%, 80%, 85%, 90%, 91%, 92%, 93%, 94%, 95%, 96%, 97%, 98% or 99% identical to the sequence as set forth in SEQ ID NO:44 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:44 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:43.

The term "BRCA1" refers to the BRCA1, DNA repair associated gene (Ensembl: ENSG00000012048; HGNC: 1100). For example, an exemplary splice variant of the gene is set forth in the nucleotide sequence as defined in NCBI Reference Sequence NM_007294.4 which encodes the coding sequence CCDS11454 or the nucleotide sequence as set forth in SEQ ID NO:45, which correspond to the sequence of the above indicated coding sequence of the BRCA1 transcript, and and encodes the corresponding amino acid sequence for example as set forth in SEQ ID NO:46, which correspond to the protein sequences defined in NCBI Protein Accession Reference Sequence NP_009229.2 encoding the BRCA1 polypeptide.

The term "BRCA1" also comprises nucleotide sequences showing a high degree of homology to BRCA1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:45 or amino acid sequences being at least 75%, 80%, 85%, 90%, 91%, 92%, 93%, 94%, 95%, 96%, 97%, 98% or 99% identical to the sequence as set forth in SEQ ID NO:46 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:46 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:45.

The term "BRCA2" refers to the BRCA2, DNA repair associated gene (Ensembl: ENSG00000139618; HGNC: 1101). For example, an exemplary splice variant of the gene is set forth in the nucleotide sequence as defined in NCBI Reference Sequence NM_000059.4 which encodes the coding sequence CCDS9344 or the nucleotide sequence as set forth in SEQ ID NO:47, which correspond to the sequence of the above indicated coding sequence of the BRCA2 transcript, and and encodes the corresponding amino acid sequence for example as set forth in SEQ ID NO:48, which correspond to the protein sequences defined in NCBI Protein Accession Reference Sequence NP_000050.3 encoding the BRCA2 polypeptide.

The term "BRCA2" also comprises nucleotide sequences showing a high degree of homology to BRCA2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:47 or amino acid sequences being at least 75%, 80%, 85%, 90%, 91%, 92%, 93%, 94%, 95%, 96%, 97%, 98% or 99% identical to the sequence as set forth in SEQ ID NO:48 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:48 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:47.

The term "CDK12" refers to the cyclin dependent kinase 12 gene (Ensembl: ENSG00000167258; HGNC: 24224). For example, an exemplary splice variant of the gene is set forth in the nucleotide sequence as defined in NCBI Reference Sequence NM_016507.4 which encodes the coding sequence CCDS11337 or the nucleotide sequence as set forth in SEQ ID NO:49, which correspond to the sequence of the above indicated coding sequence of the CDK12 transcript, and and encodes the corresponding amino acid sequence for example as set forth in SEQ ID NO:50, which correspond to the protein sequences defined in NCBI Protein Accession Reference Sequence NP_057591.2 encoding the CDK12 polypeptide.

The term "CDK12" also comprises nucleotide sequences showing a high degree of homology to CDK12, e.g., nucleic acid sequences being at least 75%, 80%, 85%, 90%, 91%, 92%, 93%, 94%, 95%, 96%, 97%, 98% or 99% identical to the sequence as set forth in SEQ ID NO:49 or amino acid sequences being at least 75%, 80%, 85%, 90%, 91%, 92%, 93%, 94%, 95%, 96%, 97%, 98% or 99% identical to the sequence as set forth in SEQ ID NO:50 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:50 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:49.

The term "FANCA" refers to the FA complementation group A gene (Ensembl: ENSG00000187741; HGNC: 3582). For example, an exemplary splice variant of the gene is set forth in the nucleotide sequence as defined in NCBI Reference Sequence NM_000135.4 which encodes the coding sequence CCDS32515 or the nucleotide sequence as set forth in SEQ ID NO:51, which correspond to the sequence of the above indicated coding sequence of the FANCA transcript, and and encodes the corresponding amino acid sequence for example as set forth in SEQ ID NO:52 which correspond to the protein sequences defined in NCBI Protein Accession Reference Sequence NP_000126.2 encoding the FANCA polypeptide.

The term "FANCA" also comprises nucleotide sequences showing a high degree of homology to FANCA, e.g., nucleic acid sequences being at least 75%, 80%, 85%, 90%, 91%, 92%, 93%, 94%, 95%, 96%, 97%, 98% or 99% identical to the sequence as set forth in SEQ ID NO:51 or amino acid sequences being at least 75%, 80%, 85%, 90%, 91%, 92%, 93%, 94%, 95%, 96%, 97%, 98% or 99% identical to the sequence as set forth in SEQ ID NO:52 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:52 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:51.

The term "MRE11" refers to the MRE11 homolog, double strand break repair nuclease gene (Ensembl: ENSG00000020922; HGNC: 7230). For example, an exemplary splice variant of the gene is set forth in the nucleotide sequence as defined in NCBI Reference Sequence NM_005591.4 which encodes the coding sequence CCDS8298 or the nucleotide sequence as set forth in SEQ ID NO:53, which correspond to the sequence of the above indicated coding sequence of the MRE11 transcript, and and encodes the corresponding amino acid sequence for example as set forth in SEQ ID NO:54, which correspond to the protein sequences defined in NCBI Protein Accession Reference Sequence NP_005581.2 encoding the MRE11 polypeptide.

The term "MRE11" also comprises nucleotide sequences showing a high degree of homology to MRE11, e.g., nucleic acid sequences being at least 75%, 80%, 85%, 90%, 91%, 92%, 93%, 94%, 95%, 96%, 97%, 98% or 99% identical to the sequence as set forth in SEQ ID NO:53 or amino acid sequences being at least 75%, 80%, 85%, 90%, 91%, 92%, 93%, 94%, 95%, 96%, 97%, 98% or 99% identical to the sequence as set forth in SEQ ID NO:54 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:54 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:53.

The term "PALB2" refers to the partner and localizer of BRCA2 gene (Ensembl: ENSG00000083093; HGNC: 26144). For example, an exemplary splice variant of the gene is set forth in the nucleotide sequence as defined in NCBI Reference Sequence NM_024675.4 which encodes the coding sequence CCDS32406 or the nucleotide sequence as set forth in SEQ ID NO:55, which correspond to the sequence of the above indicated coding sequence of the PALB2 transcript, and and encodes the corresponding amino acid sequence for example as set forth in SEQ ID NO:56, which correspond to the protein sequences defined in NCBI Protein Accession Reference Sequence NP_078951.2 encoding the PALB2 polypeptide.

The term "PALB2" also comprises nucleotide sequences showing a high degree of homology to PALB2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:55 or amino acid sequences being at least 75%, 80%, 85%, 90%, 91%, 92%, 93%, 94%, 95%, 96%, 97%, 98% or 99% identical to the sequence as set forth in SEQ ID NO:56 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:56 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:55.

The term "biological sample" or "sample obtained from a subject" refers to any biological material obtained via suitable methods known to the person skilled in the art from a subject, e.g., a prostate cancer patient. The term "prostate cancer subject" refers to a person having or suspected of having prostate cancer.

The biological sample used may be collected in a clinically acceptable manner, e.g., in a way that nucleic acids (in particular RNA) or proteins are preserved.

The biological sample(s) may include body tissue and/or a fluid, such as, but not limited to, blood, sweat, saliva, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the prostate of a male subject. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing prostate secreted exosomes, or cell lines or cancer cell line. In one particular realization, biopsy or resections samples may be obtained and/or used. Such samples may include cells or cell lysates.

Therefore in an embodiment the biological sample obtained from a subject is a biopsy. In a further preferred embodiment, the method includes providing or opbtaining a biopsy. In a preferred embodiment the biopsy is a prostate biopsy.

It is also conceivable that the content of a biological sample is submitted to an enrichment step. For instance, a sample may be contacted with ligands specific for the cell membrane or organelles of certain cell types, e.g., prostate cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

The term "prostate cancer" refers to a cancer of the prostate gland in the male reproductive system, which occurs when cells of the prostate mutate and begin to multiply out of control. Typically, prostate cancer is linked to an elevated level of prostate-specific antigen (PSA). In one embodiment of the present invention the term "prostate cancer" relates to a cancer showing PSA levels above 3.0. In another embodiment the term relates to cancer showing PSA levels above 2.0. The term "PSA level" refers to the concentration of PSA in the blood in ng/ml.

The term "non-progressive prostate cancer state" means that a sample of an individual does not show parameter values indicating "biochemical recurrence" and/or "clinical recurrence" and/or "metastases" and/or "castration-resistant disease" and/or "prostate cancer or disease specific death".

The term "progressive prostate cancer state" means that a sample of an individual shows parameter values indicating "biochemical recurrence" and/or "clinical recurrence" and/or "metastases" and/or "castration-resistant disease" and/or "prostate cancer or disease specific death".

The term "biochemical recurrence" generally refers to recurrent biological values of increased PSA indicating the presence of prostate cancer cells in a sample. However, it is also possible to use other markers that can be used in the detection of the presence or that rise suspicion of such presence.

The term "clinical recurrence" refers to the presence of clinical signs indicating the presence of tumour cells as measured, for example using in vivo imaging.

The term "metastases" refers to the presence of metastatic disease in organs other than the prostate.

The term "castration-resistant disease" refers to the presence of hormone-insensitive prostate cancer; i.e., a cancer in the prostate that does not any longer respond to androgen deprivation therapy (ADT).

The term "prostate cancer specific death or disease specific death" refers to death of a patient from his prostate cancer.

When used herein, the term "PDE4D7 KD genes" is interchangably used with "KD genes" or "knock-down genes" refers to one or more of the genes selected from ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2.

When used herein the term "response of a prostate cancer subject to radiotherapy" refers to the situation wherein radiotherapy improves or does not improve the disease status of the suibject with prostate cancer. Herein, "not improve" may refer to the situation where the radiotherapy has no significant effect or wherein the radiotherapy worsens the status of the subject. Worsening or improving of the status of the patient may refer to tumor size or mass, cancer free survival or survival. Therefore a favorable response to radiotherapy may be a decrease in tumor size or mass, increase in tumor free survival time or an overal increase in survival time for the subject. Accordingly, a non-favorable response to radiotherapy may be an increase in tumor size or mass, decrease in tumor free survival time or an overal decrease in survival time for the subject.

The method is based on the expression levels of three or more target genes selected from ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2. It is appreciated that the method may be performed on an input relating to the expression levels of the three or more genes, or determiing the expression levels may be part of the method.

It is further envisioned that the method is performed by a processor. Therefore, in an embodiment the invention relates to a computer imlpemented method of predicting a response of a prostate cancer subject to radiotherapy, comprising: receiving the result of a determination of the gene expression levels for each of three or more genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, said gene expression levels being determined in a biological sample obtained from the subject, and determining the prediction of the radiotherapy response based on the gene expression levels for the three or more genes, wherein said prediction is a favorable repsonse or a non-favorable response to radiotherapy.

It is preferred that the determining of the prediction of the radiotherapy response comprises combining the gene expression profiles for two or more, for example, 2, 3, 4, 5, 6, 7, 8, 9, 10, 11, 12, 13, 14, 15, 16, 17, 18, 19, 20, 21, 22, 23, 24, 25, 26, 27 or all, of the PDE4D7 KD genes with a regression function that had been derived from a population of prostate cancer subjects.

Therefore, in an embodiment the three or more genes comprise six or more, preferably, nine or more, most preferably, all of the genes.

Cox proportional-hazards regression allows analyzing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g., death or disease recurrence) is called the hazard. Next to the information on whether or not the tested endpoint was reached by e.g. subject in a patient cohort (e.g., patient did die or not) also the time to the endpoint is considered in the regression analysis. The hazard is modeled as: $H(t) = Ho(t) \cdot \exp(w_1 \cdot V_1 + w_2 \cdot V_2 + w_3 \cdot V_3 + ...)$, where $V_1$, $V_2$, $V_3$ ... are predictor variables and $H_0(t)$ is the baseline hazard while $H(t)$ is the hazard at any time t. The hazard ratio (or the risk to reach the event) is represented by $Ln[H(t)/ H_0(t)] = w_1 \cdot V_1 + w_2 \cdot V_2 + w_3 \cdot V_3 + ...$, where the coefficients or weights $w_1$, $w_2$, $w_3$ ... are estimated by the Cox regression analysis and can be interpreted in a similar manner as for logistic regression analysis.

In one particular realization, the prediction of the radiotherapy response is determined as follows: PDE4D7_KD_model:

$$(w_1 \cdot ACPP) + (w_2 \cdot AR) + (w_3 \cdot CDH1) + [...] + (w_{28} \cdot PALB2) \qquad (2)$$

where $w_1$ to $w_{28}$ are weights and ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2 are the expression levels of the genes.

Exemplary values for the weights $w_n$ are provided below in Table 1. It is however understood that other values may be used within the margin of error for determining the optimal constants based on the present samples. Therefore, it is envisioned that the weight may chosen such that it is within the range of the constant listed below in Table 1 plus or minus the standard error listed in the table. For example, the used w value listed for ETV1 is 0.1782 and the standard error is 0.2464, meaning that any value between -0.0682 (0.1782 - 0.2464) and 0.4246 (0.1782 + 0.2464) can be used. The same applies to the other 27 genes listed below.

TABLE 1. Weights listed per gene and standard error.

| Gene symbol | w value | Std. Error |
|---|---|---|
| ETV1 | 0,1782 | 0,2464 |
| BRCA1 | 0,2173 | 0,4273 |
| ACPP | -0,5043 | 0,318 |
| MRE11 | 1,0005 | 0,5635 |
| CDH1 | -0,2284 | 0,4041 |
| MLH1 | 0,2555 | 0,4566 |
| PALB2 | 0,6905 | 0,461 |
| FOLH1 | 1,0959 | 0,4889 |
| NRP1 | -0,7425 | 0,3503 |
| SPDEF | 0,3798 | 0,4954 |
| FOXA | 0,3105 | 0,7887 |
| EHF | -0,8092 | 0,5725 |
| BRCA2 | 0,6651 | 0,4364 |
| KLK3 | -1,3249 | 0,7382 |
| ATM | -1,6033 | 0,5829 |
| SLC45A3 | 0,2 | 0,5553 |
| HOXB13 | 2,2061 | 0,9177 |
| NKX3_1 | -0,2073 | 0,6932 |
| CDK12 | -1,6752 | 0,5085 |
| KLK2 | 1,2135 | 0,7368 |
| AR | 0,01156 | 0,4726 |
| ATR | -0,1947 | 0,3692 |
| NAALADL2 | -0,3543 | 0,3205 |
| NQO1 | -0,3 | 0,345 |
| FANCA | 0,09164 | 0,4722 |
| MAOA | -0,7141 | 0,2853 |
| MME | 0,4207 | 0,3017 |
| MYO6 | -0,03236 | 0,449 |

The prediction of the radiotherapy response may also be classified or categorized into one of at least two risk groups, based on the value of the prediction of the radiotherapy response. For example, there may be two risk groups, or three risk groups, or four risk groups, or more than four predefined risk groups. Each risk group covers a respective range of (non-overlapping) values of the prediction of the radiotherapy response. For example, a risk group may indicate a probability of occurrence of a specific clinical event from 0 to <0.1 or from 0.1 to <0.25 or from 0.25 to <0.5 or from 0.5 to 1.0 or the like.

It is further preferred that the determining of the prediction of the radiotherapy response is further based on one or more clinical parameters obtained from the subject.

As mentioned above, various measures based on clinical parameters have been investigated. By further basing the prediction of the radiotherapy response on such clinical parameter(s), it can be possible to further improve the prediction.

In an embodiment the determining of the prediction of the therapy response comprises combining the gene expression levels for three or more, for example, 3, 4, 5, 6, 7, 8, 9 or all, of the genes with a regression function that had been derived from a population of prostate cancer subjects.

In an embodiment the determining of the prediction of the radiotherapy response is further based on one or more clinical parameters obtained from the subject.

In an embodiment the clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

In an embodiment the determining of the prediction of the radiotherapy response comprises combining the gene expression levels for the three or more KD genes genes and the one or more clinical parameters obtained from the subject with a regression function that had been derived from a population of prostate cancer subjects.

It is further preferred that the gene expression profiles for the one or more PDE4D7 KD genes and the one or more clinical parameters obtained from the subject are combined with a regression function that had been derived from a population of prostate cancer subjects.

In one particular realization, the prediction of the radiotherapy response is determined as follows (PDE4D7_clinical_model):

$$(w_{29} \cdot PDE4D7\_KD\_model) + (w_{30} \cdot pGGG)$$

where $w_{29}$ and $w_{30}$ are weights, PDE4D7_KD_model is the above-described regression model based on the expression profiles for the three or more, for example, 3, 4, 6, 7, 8, 9, 10, 11, 12, 13, 14, 15, 16, 17, 18, 19, 20, 21, 22, 23, 24, 25, 26, 27 or all, of the PDE4D7 KD genes, and pGGG is the pathological Gleason grade group. In one example, $w_{29}$ may be about 0.7809 to 1.0847, such as 0.9328, and $w_{30}$ may be about 0.2534 to 0.6436, such as 0.4485.

In a further realization, the PDE4D7_clinical model may further be used to differentiate to distinguish between more risk classes. For the figures with the PDE4D7_clinical score the threshold to divide the patients into two groups is indicated in the legend of the figures. For the PDE4D7_clinical_class model the patients are split into three groups (low, intermediate, high) risk instead of only two groups. The cut-offs for the classes are based on the PDE4D7_clinical score which is calculated based on the Cox regression model: low risk (<0); intermediate risk (0-4); high risk (>4);

In an embodiment the biological sample is obtained from the subject before the start of the radiotherapy, preferably wherein the biological sample is a prostate sample or a prostate cancer sample.

In an embodiment the radiotherapy is radical radiotherapy or salvage radiotherapy.

In an embodiment a therapy is recommended or performed based on the prediction, wherein:

- if the prediction is non-favorable, the recommended therapy comprises one or more of:

    (i) radiotherapy provided earlier than is the standard;
    (ii) radiotherapy with an increased radiation dose;
    (iii) an adjuvant therapy, such as androgen deprivation therapy, preferably wherein the adjuvant therapy is a combination of androgen deprivation therapy with second line anti-androgens, radiation, chemotherapy and/or immunotherapy; and
    (iv) an alternative therapy that is not a radiation therapy.

The degree to which the prediction is negative may determine the degree to which the recommended therapy deviates from the standard form of radiotherapy.

In an embodiment a therapy is recommended based on the prediction, wherein:

- if the prediction is favorable, the recommended therapy comprises one or more of:

(v) radical radiotherapy; and
(vi) salvage radiotherapy; and
(vi) salvage radiotherapy at de-escalated dose levels; and
(vii) watchful waiting.

In a second aspect the invention relates to a computer program product comprising instructions which, when the program is executed by a computer, cause the computer to carry out a method comprising:

- receiving data indicative of a gene expression profile for each of three or more, for example, 3, 4, 5, 6, 7, 8, 9 or all, genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, said gene expression levels being determined in a biological sample obtained from a prostate cancer subject,
- determining a prediction of a response of a prostate cancer subject to therapy based on the gene expression profile(s) for the three or more genes, wherein said prediction is a favorable repsonse or a non-favorable response to radiotherapy. In an embodiment the computer program product is used to perfomr the method of the first aspect of the invnetion.

In a further aspect of the present invention, an apparatus for predicting a response of a prostate cancer subject to radiotherapy is presented, comprising:

- an input adapted to receive data indicative of a gene expression profile for each of three or more, for example, 3, 4, 5, 6, 7, 8, 9 or all, genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, said gene expression profiles being determined in a biological sample obtained from the subject,
- a processor adapted to determine the prediction of the radiotherapy response based on the gene expression profile(s) for the three or more genes, and
- optionally, a providing unit adapted to provide the prediction or a therapy recommendation based on the prediction to a medical caregiver or the subject.

In a third aspect the invention relates to a diagnostic kit, comprising:

- at least three sets of polymerase chain reaction primer pair, and optionally at least three probes, for determining the expression level for each of three or more, for example, 3, 4, 5, 6, 7, 8, 9 or all, genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, in a sample. Optionally the kit further comprises the computer program product or the apparatus as described herein. The kit may further comprise any one of buffers, dNTP, magnesium salt solution or a polymerase enzyme.

In a fourth aspect, the invention relates to the use of the kit as defined in the third aspect of the invention in a method of predicting a response of a prostate cancer subject to radiotherapy, preferably for use in the method according to the first aspect of the invention.
In a fifth aspect, the invention relates to a method, comprising:

- receiving a biological sample obtained from a prostate cancer subject,
- using the kit as defined in the third aspect of the invention to determine a gene expression profile for each of three or more, for example, 3, 4, 5, 6, 7, 8, 9 or all, genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, in the biological sample obtained from the subject.

In a fifth aspect, the invention relates to the use of a gene expression profile for each of three or more, for example, 3, 4, 5, 6, 7, 8, 9 or all, genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, in a method of predicting a response of a prostate cancer subject to radiotherapy, comprising:

- determining the prediction of the radiotherapy response based on the gene expression levels for the three or more genes, wherein said prediction is a favorable repsonse or a non-favorable response to radiotherapy.

EXAMPLES

Example 1

LNCaP cells (wild type; wt) were cultured and transfected using different lentiviral vetors with short hairpin RNA (shRNA) constructs targeting the PDE4D7 mRNA. Several clones were selected which stably expressed the shRNA-PDE4D7 construct. Using qPCR, knockdown of PDE4D7 expression was confirmed. Next RNA sequencing was performed on LNCaP wt cells and three different shRNA-PDE4D7 expressing LNCaP clones. Quality control was performred on the sequencing reads using FastQC/MultiQC. Next a trimming step was performed using SeqPurge to trim the adapater sequences from the sequencing reads. Next rRNA sequences were removed using Bowtie 2. Next, STAR was used to align the processed sequencing reads and featureCounts was used quantify reads mapped to genes. From the resulting data, transcripts per million are calculated for each gene, these numbers were log2 transformed and normalized based on reference genes. Lastly a z-score transformation was performed.

Differential gene expression between the wildtyp LNCaP and three different knockdown clones was determined. 113 differentially expressed genes were initially identified with a confidence of p<1E-20. On these genes, pathway analysis for enriched geens (DisGenNET) was performed using the terms prostatic neoplasm, malignant neoplasm of the prostate, prostate carcinoma, neoplasm metastasis and tumor progression. Based on these criteria, 20 prostate neoplasm/metastasis asociated genes and 8 DNA repair associated genes were selected, resulting in a group of 28 genes: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2. Using multi-variate Cox regression analysis a model was build for giving a PDE4D7 KD score (herein also referred to as "KD score" or knock-down score). A first cohort of 653 patient samples resulting in 572 samples for downstream analysis after quality control of the samples was used, from this first cohort samples belonging to patients undergoing salvation radiotherapy with or without androgen deprivation therapy were selected for training the model. The trained model was then validated on a second cohort of patients from which patient samples were used from patients undergoing salvage radiotherapy with or without androgen deprivation therapy. These data are depicted on the fugures as follows:

- Training on patient cohort: Figures 1-14

  - Figures 1-4: endpoint overall survival
  - Figure 5-8: metastases progression free survival
  - Figures 9-12: endpoint prostate cancer specific survival
  - Figures 13, 14: RT dose low vs high risk disease; endpoint overall survival

- Testing on 151 patient cohort: Figures 17-26

  - Figures 15-18: endpoint overall survival
  - Figure 19-22: metastases progression free survival
  - Figures 23-26: endpoint prostate cancer specific survival.

Table 2. List of differentially expressed genes in KDE4D7 knock-out clones

| ENSEMBL ID | Gene Symbol |
| --- | --- |
| ENSG00000125257 | ABCC4 |
| ENSG00000140526 | ABHD2 |
| ENSG00000014257 | ACPP |
| ENSG00000151726 | ACSL1 |
| ENSG00000205336 | ADGRG1 |
| ENSG00000132746 | ALDH3B2 |
| ENSG00000169083 | AR |

(continued)

| ENSEMBL ID | Gene Symbol |
|---|---|
| ENSG00000047648 | ARHGAP6 |
| ENSG00000006756 | ARSD |
| ENSG00000081923 | ATP8B1 |
| ENSG00000133639 | BTG1 |
| ENSG00000182795 | C1orf116 |
| ENSG00000157617 | C2CD2 |
| ENSG00000102547 | CAB39L |
| ENSG00000162545 | CAMK2N1 |
| ENSG00000162949 | CAPN13 |
| ENSG00000163814 | CDCP1 |
| ENSG00000039068 | CDH1 |
| ENSG00000181885 | CLDN7 |
| ENSG00000133313 | CNDP2 |
| ENSG00000159176 | CSRP1 |
| ENSG00000008283 | CYB561 |
| ENSG00000067048 | DDX3Y |
| ENSG00000100842 | EFS |
| ENSG00000135373 | EHF |
| ENSG00000012660 | ELOVL5 |
| ENSG00000149218 | ENDOD1 |
| ENSG00000178567 | EPM2AIP1 |
| ENSG00000006468 | ETV1 |
| ENSG00000134824 | FADS2 |
| ENSG00000166801 | FAM111A |
| ENSG00000133477 | FAM83F |
| ENSG00000145743 | FBXL17 |
| ENSG00000137460 | FHDC1 |
| ENSG00000086205 | FOLH1 |
| ENSG00000129514 | FOXA1 |
| ENSG00000164125 | GASK1B |
| ENSG00000151025 | GPR158 |
| ENSG00000083307 | GRHL2 |
| ENSG00000164116 | GUCY1A1 |
| ENSG00000184357 | H1-5 |
| ENSG00000273703 | H2BC14 |
| ENSG00000159184 | HOXB13 |
| ENSG00000261701 | HPR |
| ENSG00000080709 | KCNN2 |

(continued)

| ENSEMBL ID | Gene Symbol |
|---|---|
| ENSG00000167751 | KLK2 |
| ENSG00000142515 | KLK3 |
| ENSG00000159166 | LAD1 |
| ENSG00000136167 | LCP1 |
| ENSG00000064042 | LIMCH1 |
| ENSG00000111321 | LTBR |
| ENSG00000187398 | LUZP2 |
| ENSG00000147676 | MAL2 |
| ENSG00000189221 | MAOA |
| ENSG00000076242 | MLH1 |
| ENSG00000115648 | MLPH |
| ENSG00000196549 | MME |
| ENSG00000143158 | MPC2 |
| ENSG00000156968 | MPV17L |
| ENSG00000149573 | MPZL2 |
| ENSG00000172936 | MYD88 |
| ENSG00000196586 | MYO6 |
| ENSG00000177694 | NAALADL2 |
| ENSG00000154654 | NCAM2 |
| ENSG00000166342 | NETO1 |
| ENSG00000008441 | NFIX |
| ENSG00000167034 | NKX3-1 |
| ENSG00000181019 | NQO1 |
| ENSG00000099250 | NRP1 |
| ENSG00000198300 | PEG3 |
| ENSG00000184363 | PKP3 |
| ENSG00000067113 | PLPP1 |
| ENSG00000139220 | PPFIA2 |
| ENSG00000138814 | PPP3CA |
| ENSG00000132356 | PRKAA1 |
| ENSG00000142875 | PRKACB |
| ENSG00000184304 | PRKD1 |
| ENSG00000155066 | PROM2 |
| ENSG00000106772 | PRUNE2 |
| ENSG00000095261 | PSMD5 |
| ENSG00000132698 | RAB25 |
| ENSG00000153179 | RASSF3 |
| ENSG00000163694 | RBM47 |

(continued)

| ENSEMBL ID | Gene Symbol |
|---|---|
| ENSG00000173156 | RHOD |
| ENSG00000129824 | RPS4Y1 |
| ENSG00000111319 | SCNN1A |
| ENSG00000164300 | SERINC5 |
| ENSG00000138771 | SHROOM3 |
| ENSG00000146477 | SLC22A3 |
| ENSG00000104154 | SLC30A4 |
| ENSG00000204385 | SLC44A4 |
| ENSG00000158715 | SLC45A3 |
| ENSG00000080493 | SLC4A4 |
| ENSG00000115616 | SLC9A2 |
| ENSG00000124664 | SPDEF |
| ENSG00000166145 | SPINT1 |
| ENSG00000159674 | SPON2 |
| ENSG00000072310 | SREBF1 |
| ENSG00000149418 | ST14 |
| ENSG00000008513 | ST3GAL1 |
| ENSG00000164647 | STEAP1 |
| ENSG00000157214 | STEAP2 |
| ENSG00000132872 | SYT4 |
| ENSG00000177565 | TBL1XR1 |
| ENSG00000144115 | THNSL2 |
| ENSG00000104067 | TJP1 |
| ENSG00000151715 | TMEM45B |
| ENSG00000184012 | TMPRSS2 |
| ENSG00000142185 | TRPM2 |
| ENSG00000139865 | TTC6 |
| ENSG00000114374 | USP9Y |
| ENSG00000171940 | ZNF217 |
| ENSG00000198521 | ZNF43 |

Table 3. List of 28 selected genes ("PDE4D7 KD genes").

| GENE symbol | Gene description | ENSEMBL ID |
|---|---|---|
| ACPP | acid phosphatase, prostate [Source:HGNC Symbol;Acc:HGNC: 125] | ENSG00000014257 |
| AR | androgen receptor [Source:HGNC Symbol;Acc:HGNC:644] | ENSG00000169083 |
| CDH1 | cadherin 1 [Source:HGNC Symbol;Acc:HGNC:1748] | ENSG00000039068 |
| EHF | ETS homologous factor [Source:HGNC Symbol;Acc:H GNC :3246] | ENSG00000135373 |

(continued)

| GENE symbol | Gene description | ENSEMBL ID |
|---|---|---|
| ETV1 | ETS variant 1 [Source:HGNC Symbol;Acc:H GNC :3490] | ENSG00000006468 |
| FOLH1 | folate hydrolase 1 [Source:HGNC Symbol;Acc:H GNC:3788] | ENSG00000086205 |
| FOXA1 | forkhead box A1 [Source:HGNC Symbol;Acc:HGNC:5021] | ENSG00000129514 |
| HOXB13 | homeobox B13 [Source:HGNC Symbol;Acc:HGNC:5112] | ENSG00000159184 |
| KLK2 | kallikrein related peptidase 2 [Source:HGNC Symbol;Acc:HGNC:6363] | ENSG00000167751 |
| KLK3 | kallikrein related peptidase 3 [Source:HGNC Symbol;Acc:HGNC:6364] | ENSG00000142515 |
| MAOA | monoamine oxidase A [Source:HGNC Symbol;Acc:HGNC:6833] | ENSG00000189221 |
| MLH1 | mutL homolog 1 [Source:HGNC Symbol;Acc:HGNC:7127] | ENSG00000076242 |
| MME | membrane metalloendopeptidase [Source:HGNC Symbol;Acc:HGNC: 7154] | ENSG00000196549 |
| MYO6 | myosin VI [Source:HGNC Symbol;Acc:HGNC:7605] | ENSG00000196586 |
| NAALADL2 | N-acetylated alpha-linked acidic dipeptidase like 2 [Source:HGNC Symbol;Acc:HGNC:23219] | ENSG00000177694 |
| NKX3-1 | NK3 homeobox 1 [Source:HGNC Symbol;Acc:HGNC: 783 8] | ENSG00000167034 |
| NQO1 | NAD(P)H quinone dehydrogenase 1 [Source:HGNC Symbol;Acc:H GNC : 2874] | ENSG00000181019 |
| NRP1 | neuropilin 1 [Source:HGNC Symbol;Acc:HGNC:8004] | ENSG00000099250 |
| SLC45A3 | solute carrier family 45 member 3 [Source:HGNC Symbol;Acc:HGNC: 8642] | ENSG00000158715 |
| SPDEF | SAM pointed domain containing ETS transcription factor [Source:HGNC Symbol;Acc:HGNC: 17257] | ENSG00000124664 |
| ATM | ATM serine/threonine kinase [Source:HGNC Symbol;Acc:HGNC:795] | ENSG00000149311 |
| ATR | ATR serine/threonine kinase [Source:HGNC Symbol;Acc:HGNC:882] | ENSG00000175054 |
| BRCA1 | BRCA1, DNA repair associated [Source:HGNC Symbol;Acc:HGNC: 1100] | ENSG00000012048 |
| BRCA2 | BRCA2, DNA repair associated [Source:HGNC Symbol;Acc:HGNC:1101] | ENSG00000139618 |
| CDK12 | cyclin dependent kinase 12 [Source:HGNC Symbol;Acc:H GNC :24224] | ENSG00000167258 |
| FANCA | FA complementation group A [Source:HGNC Symbol;Acc:HGNC:3582] | ENSG00000187741 |
| MRE11 | MRE11 homolog, double strand break repair nuclease [Source:HGNC Symbol;Acc:HGNC:7230] | ENSG00000020922 |
| PALB2 | partner and localizer of BRCA2 [Source:HGNC Symbol;Acc:HGNC: 26144] | ENSG00000083093 |

TPM gene expression values

For each gene a TPM (transcript per million) expression value was calculated based on the following steps:

1. Divide the read counts derived from the RNAseq fastq raw data after mapping and alignment to the human genome by the length of each gene in kilobases. This results in reads per kilobase (RPK).
2. Sum up all RPK values in a samples and divide this number by 1,000,000. This results in a 'per million' scaling factor.
3. Divide the RPK values by the "per million" scaling factor. This results in a TPM expression value for each gene.

The TPM value per gene was used to calculate the reference normalized gene expression of each gene.

Normalized gene expression values

For the Cox regression modeling all gene TPM (transcript per million) based expression values from the RNAseq data were log2 normalized by the following transformation:

$$TPM\_log2 = log2(TPM+1) \tag{4}$$

In a second step of normalization of the TPM_log2 expression values were normalized against the mean average of four reference genes (mean(ref_genes)) as follows:

$$TPM\_log2\_norm = log2(TPM+1) - log2(mean(ref\_genes)) \tag{5}$$

The following reference genes were considered (TABLE 2):

TABLE 4: Reference genes.

| Gene Symbol | Ensembl_ID |
| --- | --- |
| ALAS1 | ENSG00000023330 |
| ACTB | ENSG00000075624 |
| RPLP0 | ENSG00000089157 |
| TBP | ENSG00000112592 |
| TUBA1B | ENSG00000123416 |
| PUM1 | ENSG00000134644 |
| YWHAZ | ENSG00000164924 |
| HPRT1 | ENSG00000165704 |
| B2M | ENSG00000166710 |
| POLR2A | ENSG00000181222 |

For these reference genes, we selected the following four B2M, HPRT1, POLR2A, and PUM1 in order to calculate the:

$$log2(mean(ref\_genes)) = AVERAGE((log2(B2M+1), log2(HPRT1+1),$$
$$log2(POLR2A+1), log2(PUM1+1)) \tag{6}$$

where the input data for the reference genes is their RNAseq measured gene expression in TPM (transcript per million), and AVERAGE is the mathematical mean.

For the multivariate analysis of the genes of interest we used the reference gene normalized log2(TPM) value of each gene as input.

Cox Regression Analysis

We then set out to test whether the combination of these twentyeight genes will exhibit more prognostic value. With Cox regression we modelled the expression levels of the twentyeight genes to prostate cancer specific death after post-surgical salvage RT either with (PDE4D7_clinical_model) or without (PDE4D7_KD_model) the presence of the variable pathological Gleason grade group (pGGG) in a cohort of 571 prostate cancer patients. We tested the two models in ROC curve analysis (data not shown) as well as in Kaplan-Meier survival analysis.

The Cox regression function was derived as follows:

PDE4D7_KD_model:

$$(w_1 \cdot ACPP) + (w_2 \cdot AR) + (w_3 \cdot CDH1) + (w_4 \cdot EHF) + (w_5 \cdot ETV1) +$$
$$(w_6 \cdot FOLH1) + (w_7 \cdot FOXA1) + (w_8 \cdot HOXB13) + (w_9 \cdot KLK2) + (w_{10}$$
$$\cdot KLK3) + (w_{11} \cdot MAOA) + (w_{12} \cdot MLH1) + (w_{13} \cdot MME) + (w_{14} \cdot$$
$$MYO6) + (w_{15} \cdot NAALADL2) + (w_{16} \cdot NKX3\text{-}1) + (w_{17} \cdot NQO1) + (w_{18}$$
$$\cdot NRP1) + (w_{19} \cdot SLC45A3) + (w_{20} \cdot SPDEF) + (w_{21} \cdot ATM) + (w_{22} \cdot$$
$$ATR) + (w_{23} \cdot BRCA1) + (w_{24} \cdot BRCA2) + (w_{25} \cdot CDK12) + (w_{26} \cdot$$
$$FANCA) + (w_{27} \cdot MRE11) + (w_{28} \cdot PALB2)$$

PDE4D7_clinical_model:

$$(w_{29} \cdot PDE4D7\_KD\_model) + (w_{30} \cdot pGGG)$$

For the PDE4D7_clinical_class model the patients are split into three groups (low, intermediate, high) risk instead of only two groups. The cut-offs for the classes are based on the PDE4D7_clinical score which is calculated based on the Cox regression model: low risk (<0); intermediate risk (0-4); high risk (>4);

The details for the weights $w_1$ to $w_{28}$ are shown in the following TABLE 1.

ROC Curve Analysis

Next, we tested the Cox regression model as outlined above for their power to predict 5-year prostate cancer specific death (PCa Death) after start of salvage radiation therapy (SRT) due to post-surgical disease recurrence. The performance of the model was compared to the EAU-BCR risk groups (see Tilki D. et al., "External validation of the European Association of Urology Biochemical Recurrence Risk groups to predict metastasis and mortality after radical prostatectomy in a European cohort", Eur Urol, Vol. 75, No. 6, pages 896-900, 2019) and to the pathological Gleason grade group (pGGG).

Kaplan-Meier Survival Analysis

For Kaplan-Meier survival curve analysis, the Cox functions of the risk models (PDE4D7_KD_model and PDE4D7_clinical_model) were categorized into two sub-cohorts based on a cut-off. The threshold for group separation into low risk and high risk was based on the mean output value of the PDE4D7_KD_model and of the PDE4D7_clinical_model, respectively, as calculated by use of the Cox regression model per patient in the entire cohort.

The patient classes represent an increasing risk to experience the tested clinical endpoints of prostate cancer specific death (PCa Death) after the start of salvage radiation therapy (SRT) due to post-surgical disease recurrence (Figs. 9 to 12, 23 to 26) or death (Death) after the start of salvage radiation therapy (SRT) due to post-surgical disease recurrence (Figs. 1 to 4, 15 to 18) or occurrence or recurrence of a metastasis (Metastasis) due to post-surgical disease recurrence (Figs 5 to 8, 19 to 22) for the two created risk models (PDE4D7_KD_model; PDE4D7_clinical_model).

Example 2

LNCaP clone FGC (ATCC® CRL1740™) cells were cultured in RPMI1640 medium supplemented with fetal bovine serum to a final concentration of 10%. For transduction with lentiviruses cells were seeded in 6-well plates at concentration 0.2x106 cell per well in 2 ml growth medium. When cells reached confluency ~30-40% they were infected with lentiviruses at MOI=10 in the presence of 10 ug/ml Polybrene. Cells were incubated with lentivirus for ~16 hours (overnight), and then medium changed. 48 hours after infection puromycin was added to the final concentration 2 ug/ml. Medium change was done every 3-4 days. After 7 days of treatment with puromycin cells were transferred from 6-well plate to 10 cm dishes for single cell colony selection (also in the presence of puromycin). Selected colonies were transferred into separate wells in 6-well plates. Upon reaching 80% confluence, cells were detached by trypsin and seeded in larger vessels (e.g. 10-cm cell culture dishes) for expansion.

RNA from cells was extracted using RNeazy kit (Qiagen). cDNA was synthesized using either oligo-dT or specific primers. qPCR was done using PrimeTime Gene Expression Master Mix (IDT, Cat. 1055772).

Several PDE4D7-shRNA construct were tested and evaluated. The construct represented with SEQ ID NO: 57 and having the sequence:

gatccgGAAATACCTGTGATTTGCTTTCTCAAGAGGAAAGCAAATCACAGGTATTTCttttttg was used for all experiments.

Discussion

The effectiveness of both radical RT and SRT for localized prostate cancer is limited, resulting in disease progression and ultimately death of patients, especially for those at high risk of recurrence. The prediction of the therapy outcome is very complicated as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the response to radical RT and to SRT, in order to increase the success rate of these therapies.

We have identified molecules of which expression shows a significant relation to mortality after radical RT and SRT and therefore are expected to improve the prediction of the effectiveness of these treatments. An improved prediction of effectiveness of RT for each patient be it in the radical or the salvage setting, will improve therapy selection and potentially survival. This can be achieved by 1) optimizing RT for those patients where RT is predicted to be effective (e.g. by dose escalation or a different starting time) and 2) guiding patients where RT is predicted not to be effective to an alternative, potentially more effective form of treatment. Further, this would reduce suffering for those patients who would be spared ineffective therapy and would reduce cost spent on ineffective therapies.

Other variations to the disclosed realizations can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

One or more steps of the method illustrated in Fig. 1 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the steps descirbed herein can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

**Claims**

1. A method of predicting a response of a prostate cancer subject to radiotherapy, comprising:

   - determining or receiving the result of a determination of the gene expression levels for each of three or more genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, said gene expression levels being determined in a biological sample obtained from the subject,
   - determining the prediction of the radiotherapy response based on the gene expression levels for the three or more genes, wherein said prediction is a favorable repsonse or a non-favorable response to radiotherapy.

2. The method according to claim 1, wherein the three or more genes comprise six or more, preferably, nine or more, most preferably, all of the genes.

3. The method according to claim 1 or 2, wherein the determining of the prediction of the therapy response comprises combining the gene expression levels for three or more, for example, 3, 4, 5, 6, 7, 8, 9 or all, of the genes with a regression function that had been derived from a population of prostate cancer subjects.

4. The method according to any one of the preceding claims, wherein the determining of the prediction of the radiotherapy response is further based on one or more clinical parameters obtained from the subject.

5. The method according to claim 4, wherein the clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

6. The method as defined in claim 4 or 5, wherein the determining of the prediction of the radiotherapy response comprises combining the gene expression levels for the three or more genes and the one or more clinical parameters obtained from the subject with a regression function that had been derived from a population of prostate cancer subjects.

7. The method according to any one of the preceding claims, wherein the biological sample is obtained from the subject before the start of the radiotherapy, preferably wherein the biological sample is a prostate sample or a prostate cancer sample.

8. The method according to any one of the preceding claims, wherein the radiotherapy is radical radiotherapy or salvage radiotherapy.

9. The method according to any one of the preceding claims, wherein a therapy is recommended based on the prediction, wherein:

- if the prediction is non-favorable, the recommended therapy comprises one or more of:

(i) radiotherapy provided earlier than is the standard;
(ii) radiotherapy with an increased radiation dose;
(iii) an adjuvant therapy, such as androgen deprivation therapy, preferably wherein the adjuvant therapy is a combination of androgen deprivation therapy with second line anti-androgens, radiation, chemotherapy and/or immunotherapy; and
(iv) an alternative therapy that is not a radiation therapy.

10. The method according to any one of the preceding claims, wherein a therapy is recommended based on the prediction, wherein:

- if the prediction is favorable, the recommended therapy comprises one or more of:

(v) radical radiotherapy; and
(vi) salvage radiotherapy; and
(vi) salvage radiotherapy at de-escalated dose levels; and
(vii) watchful waiting.

11. A computer program product comprising instructions which, when the program is executed by a computer, cause the computer to carry out a method comprising:

- receiving data indicative of a gene expression profile for each of three or more, for example, 3, 4, 5, 6, 7, 8, 9 or all, genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, said gene expression levels being determined in a biological sample obtained from a prostate cancer subject,
- determining a prediction of a response of a prostate cancer subject to therapy based on the gene expression profile(s) for the three or more genes, wherein said prediction is a favorable repsonse or a non-favorable response to radiotherapy.

12. A diagnostic kit, comprising:

- at least three sets of polymerase chain reaction primer pair, and optionally at least three probes, for determining the expression level for each of three or more, for example, 3, 4, 5, 6, 7, 8, 9 or all, genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, in a sample.

13. Use of the kit as defined in claim 12 in a method of predicting a response of a prostate cancer subject to radiotherapy, preferably for use in the method as defined in any one of claims 1 to 9.

14. A method, comprising:

- receiving a biological sample obtained from a prostate cancer subject,
- using the kit as defined in claim 12 to determine a gene expression profile for each of three or more, for example, 3, 4, 5, 6, 7, 8, 9 or all, genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, in the biological sample obtained from the subject.

15. Use of a gene expression profile for each of three or more, for example, 3, 4, 5, 6, 7, 8, 9 or all, genes selected from the group consisting of: ACPP, AR, CDH1, EHF, ETV1, FOLH1, FOXA1, HOXB13, KLK2, KLK3, MAOA, MLH1, MME, MYO6, NAALADL2, NKX3-1, NQO1, NRP1, SLC45A3, SPDEF, ATM, ATR, BRCA1, BRCA2, CDK12, FANCA, MRE11 and PALB2, in a method of predicting a response of a prostate cancer subject to radiotherapy, comprising:

- determining the prediction of the radiotherapy response based on the gene expression levels for the three or more genes, wherein said prediction is a favorable repsonse or a non-favorable response to radiotherapy.

**Fig. 1**

Fig. 2

**Fig. 3**

**Fig. 4**

Fig. 5

**Fig. 6**

Fig. 7

**Fig. 8**

Fig. 9

**Fig. 10**

**Fig. 11**

**Fig. 12**

**Fig. 13**

**Fig. 14**

Fig. 15

**Fig. 16**

Fig. 17

Death_class

EP 4 124 661 A1

**Fig. 18**

Fig. 19

Fig. 20

EP 4 124 661 A1

**Fig. 21**

Fig. 22

Metastases_class

logrank p=0,1
HR (intermed vs low)=2,4 [95% CI 1,2-4,9]
HR (high vs low)=1,9 [95% CI 0,7-5,6]
HR (high vs intermed)=0,8 [95% CI 0,3-2,2]

LNCaP_4D7_Clinical
— Low Risk
— Intermediate Risk
— High Risk

Time SRT Start to PCa Death [months]
pGleason Grade Group>1

Number at risk
Group: Low Risk

| 33 | 31 | 31 | 30 | 27 | 21 | 13 | 8 | 6 | 6 | 4 | 2 | 0 |
|----|----|----|----|----|----|----|---|---|---|---|---|---|

Group: Intermediate Risk

| 78 | 72 | 69 | 65 | 53 | 35 | 24 | 15 | 7 | 2 | 1 | 0 | 0 |
|----|----|----|----|----|----|----|----|---|---|---|---|---|

Group: High Risk

| 20 | 20 | 19 | 16 | 14 | 7 | 5 | 3 | 0 | 0 | 0 | 0 | 0 |
|----|----|----|----|----|---|---|---|---|---|---|---|---|

EP 4 124 661 A1

Fig. 23

EP 4 124 661 A1

PCa_Death_class

logrank p=0,02
HR=3,6 [95% CI 1,2-10,2]

LNCaP_4D7_kd
—— <=threshold (0)
—— >threshold (0)

Time Start SRT to PCa Death [months]
SRT_class=1

Number at risk
Group: <=threshold (0)

| | | | | | | | | | | | | | |
|---|---|---|---|---|---|---|---|---|---|---|---|---|---|
| 54 | 54 | 49 | 45 | 33 | 22 | 14 | 12 | 10 | 8 | 5 | 3 | 1 | 0 |

Group: >threshold (0)

| | | | | | | | | | | | | | |
|---|---|---|---|---|---|---|---|---|---|---|---|---|---|
| 97 | 88 | 81 | 73 | 62 | 42 | 33 | 19 | 11 | 5 | 3 | 1 | 0 | 0 |

Fig. 24

PCa_Death_class

SRT_class=1

Time Start SRT to PCa Death [months]

LNCaP_4D7_Clinical
— <=threshold (1)
— >threshold (1)

logrank p=0,04
HR=3,0 [95% CI 1,1-8,5]

Number at risk
Group: <=threshold (1)
57   56   53   48   34   26   18   13   11   9   5   3   1   0
Group: >threshold (1)
94   86   77   70   61   38   29   18   10   4   3   1   0   0

Fig. 25

Fig. 26

EP 4 124 661 A1

PCa_Death_class

pGleason Grade Group>1

Number at risk
Group: Low Risk

33 33 32 29 22 16 10 7 6 6 3 1 0

Group: Intermediate Risk

81 76 66 59 50 35 26 17 11 5 3 1 0

Group: High Risk

22 18 17 16 15 8 7 4 1 0 0 0 0

LNCaP wt (clone FCG)

↓

Lenti-viral transfection with shRNAPDE4D7 LNCaP wt (clone FCG)

↓

Selection of multiple shRNA-PDE4D7 positive LNCaP clones

↓

qPCR for PDE4D7 knock-down compared to the LNCaP wt cell line to confirm PDE4D7 knock-down

↓

RNAseq of LNCaP wt and three shRNA-PDE4D7 knock-down cell lines

→

- QC (FastQC/MultiQC)
- Trimming (SeqPurge)
- rRNA removal (Bowtie2)
- Read alignment (STAR)
- Read quant (featureCounts)
- TPM calculation
- Log2 transformation
- Ref gene normalization
- Z-score transformation

↓

Differential Gene Expression LNCaP wt vs three shRNA-PDE4D7 kd clones

↓

Identification of 113 differentially expression genes with p<1E-20

Fig. 27

Pathway analysis for enriched disease genes (DisGeNET)

Selection of 20 prostate neoplasm / metastasis related genes

Selection of 8 DNA repair related genes

Multi-variate Cox regression analysis

Prostate cancer PDE4D7 KD (clinical) score

**Fig. 28**

**Fig. 29**

Europäisches
Patentamt

European
Patent Office

Office européen
des brevets

# EUROPEAN SEARCH REPORT

Application Number

EP 21 18 7665

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/141986 A1 (SPETZLER DAVID [US] ET AL) 22 May 2014 (2014-05-22) <br> * paragraphs [0102], [0894], [0902], [0969], [1106], [1131], [1281] – [1282] * <br> * figure 65 * | 1-15 | INV. <br> C12Q1/6886 |
| X | US 2017/152571 A1 (STONE STEVEN [US] ET AL) 1 June 2017 (2017-06-01) <br> * claims 1-63 * <br> * examples 5,9 * <br> * table y * <br> * paragraphs [0140] – [0189] * | 1-15 | |
| X <br><br> Y | US 2014/220580 A1 (BROWN KIRK [US] ET AL) 7 August 2014 (2014-08-07) <br> * example 44; table 31 * <br> * paragraphs [0020] – [0021], [0319], [0384], [0528], [0581] – [0583] * <br> * figure 3 * | 1,2,7-15 <br><br> 3-6 | |
| Y | US 2019/218621 A1 (DAVICIONI ELAI [US] ET AL) 18 July 2019 (2019-07-18) <br> * claims 1-30 * | 1-15 | TECHNICAL FIELDS SEARCHED (IPC) <br><br> C12Q |
| Y | WO 2010/089707 A1 (YEDA RES & DEV [IL]; MEDICAL RES INFRASTRUCTURE AND [IL] ET AL.) 12 August 2010 (2010-08-12) <br> * claims 1-27 * <br> * page 23, paragraph 4 – page 26, paragraph 2 * <br> * example 2 * | 1-15 | |
| Y | US 2020/308654 A1 (HOFFMANN RALF DIETER [DE]) 1 October 2020 (2020-10-01) <br> * claims 1-16 * <br> * paragraphs [0023], [0046], [0104], [0141] * | 1-15 | |

-/--

The present search report has been drawn up for all claims

| Place of search | Date of completion of the search | Examiner |
|---|---|---|
| The Hague | 16 December 2021 | Bruma, Anja |

Europäisches
Patentamt

European
Patent Office

Office européen
des brevets

# EUROPEAN SEARCH REPORT

**Application Number**

EP 21 18 7665

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | MARCIA ALVES DE INDA ET AL: "Validation of Cyclic Adenosine Monophosphate Phosphodiesterase-4D7 for its Independent Contribution to Risk Stratification in a Prostate Cancer Patient Cohort with Longitudinal Biological Outcomes", EUROPEAN UROLOGY FOCUS, 1 June 2017 (2017-06-01), XP055481303, NL ISSN: 2405-4569, DOI: 10.1016/j.euf.2017.05.010 * the whole document * ----- | 1-15 | |

**TECHNICAL FIELDS SEARCHED (IPC)**

The present search report has been drawn up for all claims

| Place of search | Date of completion of the search | Examiner |
|---|---|---|
| The Hague | 16 December 2021 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 21 18 7665

This annex lists the patent family members relating to the patent documents cited in the above-mentioned European search report.
The members are as contained in the European Patent Office EDP file on
The European Patent Office is in no way liable for these particulars which are merely given for the purpose of information.

16-12-2021

| Patent document cited in search report | | Publication date | Patent family member(s) | | Publication date |
|---|---|---|---|---|---|
| US 2014141986 | A1 | 22-05-2014 | AU | 2012220872 A1 | 12-09-2013 |
| | | | CA | 2827894 A1 | 30-08-2012 |
| | | | CN | 103492590 A | 01-01-2014 |
| | | | EP | 2678448 A1 | 01-01-2014 |
| | | | JP | 2014507160 A | 27-03-2014 |
| | | | US | 2014141986 A1 | 22-05-2014 |
| | | | WO | 2012115885 A1 | 30-08-2012 |
| US 2017152571 | A1 | 01-06-2017 | CA | 2947624 A1 | 19-11-2015 |
| | | | EP | 3143160 A1 | 22-03-2017 |
| | | | EP | 3623482 A1 | 18-03-2020 |
| | | | US | 2017152571 A1 | 01-06-2017 |
| | | | WO | 2015175692 A1 | 19-11-2015 |
| US 2014220580 | A1 | 07-08-2014 | AU | 2012271516 A1 | 23-01-2014 |
| | | | BR | 112013032232 A2 | 20-09-2016 |
| | | | CA | 2839530 A1 | 20-12-2012 |
| | | | CN | 103797131 A | 14-05-2014 |
| | | | EP | 2721179 A2 | 23-04-2014 |
| | | | JP | 2014519340 A | 14-08-2014 |
| | | | KR | 20140072014 A | 12-06-2014 |
| | | | US | 2014220580 A1 | 07-08-2014 |
| | | | WO | 2012174282 A2 | 20-12-2012 |
| US 2019218621 | A1 | 18-07-2019 | AU | 2017315425 A1 | 18-04-2019 |
| | | | CN | 110506127 A | 26-11-2019 |
| | | | EP | 3504348 A1 | 03-07-2019 |
| | | | US | 2019218621 A1 | 18-07-2019 |
| | | | WO | 2018039490 A1 | 01-03-2018 |
| WO 2010089707 | A1 | 12-08-2010 | NONE | | |
| US 2020308654 | A1 | 01-10-2020 | CN | 111742061 A | 02-10-2020 |
| | | | EP | 3502280 A1 | 26-06-2019 |
| | | | EP | 3728640 A1 | 28-10-2020 |
| | | | JP | 2021506326 A | 22-02-2021 |
| | | | US | 2020308654 A1 | 01-10-2020 |
| | | | WO | 2019122037 A1 | 27-06-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BRAY F et al.** Global cancer statistics 2018: GLOBOCAN estimates of incidence and mortality worldwide for 36 cancers in 185 countries. *CA Cancer J Clin,* 2018, vol. 68 (6), 394-424
- Cancer Facts & Figures 2010. ACS (American Cancer Society), 2010
- **GRIMM P et al.** Comparative analysis of prostate-specific antigen free survival outcomes for patients with low, intermediate and high risk prostate cancer treatment by radical therapy. Results from the Prostate Cancer Results Study Group. *BJU Int,* 2012, (1), 22-29
- **DAL PRA A et al.** Contemporary role of postoperative radiotherapy for prostate cancer. *Transl Androl Uro,* 2018, vol. 7 (3), 399-413
- **HERRERA F.G. ; BERTHOLD D.R.** Radiation therapy after radical prostatectomy: Implications for clinicians. *Front Oncol,* 2016, vol. 6 (117
- **PISANSKY T.M. et al.** Salvage radiation therapy dose response for biochemical failure of prostate cancer after prostatectomy - A multi-institutional observational study. *Int J Radiat Oncol Biol Phys,* 2016, vol. 96 (5), 1046-1053
- **RESNICK M.J. et al.** Long-term functional outcomes after treatment for localized prostate cancer. *N Engl J Med,* 2013, vol. 368 (5), 436-445
- **HEGARTY S.E. et al.** Radiation therapy after radical prostatectomy for prostate cancer: Evaluation of complications and influence of radiation timing on outcomes in a large, population-based cohort. *PLoS One,* 2015, vol. 10 (2

- **PARAVATI A.J. et al.** Variation in the cost of radiation therapy among medicare patients with cancer. *J Oncol Pract,* 2015, vol. 11 (5), 403-409
- **HALL W.A. et al.** Biomarkers of outcome in patients with localized prostate cancer treated with radiotherapy. *Semin Radiat Oncol,* 2016, vol. 27, 11-20
- **RAYMOND E et al.** An appraisal of analytical tools used in predicting clinical outcomes following radiation therapy treatment of men with prostate cancer: A systematic review. *Radiat Oncol,* 2017, vol. 12 (1), 56
- **HENDERSON D.J. et al.** The cAMP phosphodiesterase-4D7 (PDE4D7) is downregulated in androgen-independent prostate cancer cells and mediates proliferation by compartmentalizing cAMP at the plasma membrane of VCaP prostate cancer cells. *Br J Cancer,* 2014, vol. 110 (5), 1278-1287
- **ALVES DE INDA M et al.** Validation of Cyclic Adenosine Monophosphate Phosphodiesterase-4D7 for its Independent Contribution to Risk Stratification in a Prostate Cancer Patient Cohort with Longitudinal Biological Outcomes. *Eur Urol Focus,* 2018, vol. 4 (3), 376-384
- **TILKI D et al.** External validation of the European Association of Urology Biochemical Recurrence Risk groups to predict metastasis and mortality after radical prostatectomy in a European cohort. *Eur Urol,* 2019, vol. 75 (6), 896-900